# EUROPÄISCHE PATENTSCHRIFT

(11) **EP 1 907 350 B1**
(45) Veröffentlichungstag und Bekanntmachung des Hinweises auf die Patenterteilung: **11.01.2012**
(21) Anmeldenummer: 06776326.8
(22) Anmeldetag: 20.07.2006
(51) Int. Cl.: C07C 215/64, A61K 31/135

(54) **SALZ VON FUMARSÄURE UND 3- (2-DIMETHYLAMINO) METHYL- ( CYCLOHEX-1-YL) ) PHENOL UN DESSEN KRISTALLINE FORMEN**
SALT OF FUMARIC ACID AND 3-(2-DIMETHYLAMINO)METHYL(CYCLOHEX-1-YL))PHENOL AND ITS CRYSTALLINE FORMS
SEL D'ACIDE FUMARIQUE ET 3-(2-DIMETHYLAMINO)METHYLE-(CYCLOHEX-1-YLE) ) PHENOL ET SES FORMES CRISTALLINES

(30) Priorität: 22.07.2005 DE 102005034973
(43) Veröffentlichungstag der Anmeldung: 09.04.2008
(73) Patentinhaber: Grünenthal GmbH, 52078 Aachen (DE)
(72) Erfinder: GRUSS, Michael, 52080 Aachen (DE); HELL, Wolfgang, 52066 Aachen (DE); SZELAGIEWICZ, Martin, CH-4142 Münchenstein (CH); BERGHAUSEN, Jörg, 79541 Lörrach (DE); DE PAUL, Susan, Margaret, CH-8057 Zürich (CH); VON RAUMER, Markus, CH-4144 Arlesheim (CH)
(86) Internationale Anmeldenummer: PCT/EP2006/007161
(87) Internationale Veröffentlichungsnummer: WO 2007/009793

(56) Entgegenhaltungen:
- EP-A1- 0 753 506

## Beschreibung

Die vorliegende Erfindung betrifft ein Salz von Fumarsäure und 3-[2-(Dimethylamino)methyl-(cyclohex-1-yl)]-phenol in einer Zusammensetzung von 1:1, stabile kristalline Formen des Salzes sowie Verfahren zu deren Herstellung, eine pharmazeutische Zusammensetzung und die Verwendung des Salzes als pharmazeutischer Wirkstoff in einer Zusammensetzung.

In der EP-A1-0 753 506 werden 3-[2-(Dimethylamino)methyl-(cyclohex-1-yl)]-phenole mit analgetischer Wirkung beschrieben. Im Text wird erwähnt, dass man auch Salze aus den freien Basen herstellen kann, wobei auch Fumarsäure als mögliches Anion erwähnt wird. In den Beispielen werden ausschliesslich Hydrochloride bereitgestellt, also ein Salz mit monovalentem Anion. Die EP-A1-0 753 506 enthält keinerlei Hinweise, in welcher Stöchiometrie 3-[2-(Dimethylamino)methyl-(cyclohex-1-yl)]-phenole mit bivalenten Anionen vorliegen können, zum Beispiel als Hemi- oder 1:1-Salze. Die nähere Untersuchung von 3-[2-(Dimethylamino)methyl-(cyclohex-1-yl)]-phenol-hydrochlorid hat ergeben, dass sich diese kristalline Festsubstanz durch einen ausgeprägten Polymorphismus auszeichnet und mehrere kristalline, auch metastabile Formen bildet. Zusätzlich neigt dieses Hydrochlorid stark zur Bildung von Hydraten und Solvaten, was für die gezielte Herstellung einer spezifischen kristallinen Form ein erheblicher Nachteil ist. Das kristalline 3-[2-(Dimethylamino)meihyl-(cyclohex-1-yl)]-phenol-hydrochlorid hat sich zudem als ausgesprochen hygroskopisch erwiesen. Dieses Eigenschaftsprofil von 3-[2-(Dimethylamino)methyl-(cyclohex-1-yl)]-phenol-hydrochlorid macht deutlich, dass es sehr schwierig ist, mit dieser Wirksubstanz pharmazeutische Zusammensetzungen mit reproduzierbaren Eigenschaften bereitzustellen, die auch über den Zeitraum einer Lagerung erhalten bleiben. Für die Erreichung dieser Ziele wären zumindest aufwendige Schutzmassnahmen erforderlich.

Es wurde nun überraschend gefunden, dass 3-[2-(Dimethylamino)methyl-(cyclohex-1-yl)]-phenol mit Fumarsäure ein Fumarat als kristalline Festsubstanz bildet, in einer Zusammensetzung im Verhältnis 1:1 von Fumarat und 3-[2-(Dimethylamino)methyl-(cyclohex-1-yl)]-phenol. Es wurde ferner überraschend gefunden, dass Fumarat nicht hygroskopisch ist, an Luft stabil ist und keine Hydrate beziehungsweise Solvate bildet. Es wurde auch überraschend gefunden, dass 3-[2-(Dimethylamino)methyl-(cyclohex-1-yl)]-phenol-Fumarat nur wenige bei niedriger beziehungsweise bei höherer Temperatur stabile Formen zu bilden vermag, wobei Form B die bei Raumtemperatur stabile Form ist. Die bei höherer Temperatur stabile Form A kann ebenfalls in Form B umgewandelt werden, wobei beide Formen bei niedriger Temperatur in Mischung vorliegen können. Die kristalline Form B zeichnet sich auch durch eine hohe chemische Beständigkeit bei Temperaturen unter 100 °C aus. Ferner weist 3-[2-(Dimethylamino)methyl-(cyclohex-1-yl)]-phenol-Fumarat auch wertvolle biologische Eigenschaften, wie zum Beispiel gute Löslichkeit besonders in polaren und protischen Lösungsmitteln einschliesslich Wasser, und eine gute Bioverfügbarkeit auf. 3-[2-(Dimethylamino)methyl-(cyclohex-1-yl)]-phenol-Fumarat eignet sich auf Grund seines Eigenschaftsprofils sehr gut zur Formulierung von pharmazeutischen Zusammensetzungen.

Ein erster Gegenstand der Erfindung sind Salze der Fumarsäure mit 3-[2-(Dimethylamino)methyl-(cyclohex-1-yl)]-phenol der Formel I

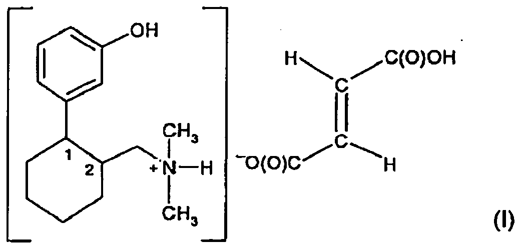

Die erfindungsgemäßen Verbindungen der Formel I enthalten in den 1- und 2-Positionen des Cyclohexanringes je ein chirales C-Atom. Die erfindungsgemäßen Verbindungen der Formel I umfassen alle Stereoisomeren und Gemische von Stereoisomeren. Bevorzugt sind Diastereomere oder Gemische von enantiomeren Diastereomeren mit Transkonfiguration des Phenylringes und der Dimethylaminomethylgruppe (1R,2R- beziehungsweise 1 S,2S-Konfiguration), wobei das Enantiomer mit der absoluten Konfiguration (1 R,2R) ganz besonders bevorzugt ist.

Die Struktur des (1 R,2R)-Enantiomers von 3-[2-(Dimethylamino)methyl-(cyclohex-1-yl)]-phenols ist nachstehend wiedergegeben:

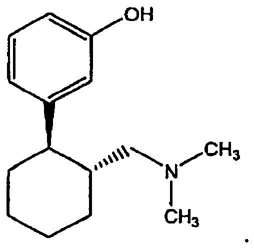

Die Verbindungen der Formel I können, analog zu den in der EP-A1-0 753 506 allgemein für die Herstellung von Salzen beschriebenen Verfahren, aus der freien

Base und Umsetzung mit Fumarsäure in Gegenwart von Wasser erhalten werden. Die freie Base 3-[2-(Dimethylamino)methyl-(cyclohex-1-yl)]-phenol kann zum Beispiel aus dem in den Beispielen 9 und 10 der EP-A1-0 753 506 beschriebenen Hydrochlorid isoliert werden. Hierzu löst man das Hydrochlorid in einem organischen Lösungsmittel, versetzt mit einer wässrigen anorganischen Base, zum Beispiel Alkalimetallbasen oder auch Alkalimetallhydrogencarbonaten (wie LiOH, NaOH, KOH, NaHCO₃ und KHCO₃), und trennt die organische Phase ab. Die organische Phase kann getrocknet werden und entweder die Base in üblicher Weise isoliert oder gegebenenfalls nach einer Konzentrierung durch Verdampfen vom Lösungsmittel direkt zur Salzbildung verwendet werden.

Ein weiterer Gegenstand der Erfindung ist ein Verfahren zur Herstellung von 3-[2-(Dimethylamino)methyl-(cyclohex-1-yl)]-phenol-fumarat der Formel I, umfassend die Vereinigung von 3-[2-(Dimethylamino)methyl-(cyclohex-1-yl)]-phenol und Fumarsäure, wobei vorzugsweise wenigstens eine der Komponenten in gelöster oder suspendierter Form vorliegt.

Ein weiterer Gegenstand der Erfindung ist ein Verfahren zur Herstellung von 3-[2-(Dimethy(amino)methyl-(cyclohex-1-yl)]-phenol-Fumarat der Formel I, umfassend die Schritte
a) Lösen oder Suspendieren von 3-[2-(Dimethylamino)methyl-(cyclohex-1-yl)]-phenol in einem Lösungsmittel, oder Vorlegen von 3-[2-(Dimethylamino)methyl-(cyclohex-1-yl)]-phenol in Substanz,
b) Vermischen der Lösung, Suspension oder Substanz mit Fumarsäure, einer Suspension oder einer Lösung von Fumarsäure in einem Lösungsmittel, gegebenenfalls Abkühlen und Halten bei niedriger Temperatur,
c) Isolieren der Verbindung der Formel I,
wobei vorzugsweise in keiner Verfahrensstufe die Temperatur über 90 °C liegt und die Schritte a) und b) auch vertauscht sein können.

Es wurde überraschend gefunden, dass die Salzbildung dann zu nur einer kristallinen Form führt, nämlich Form B, wenn man die Temperatur kontrolliert und keine Temperaturen von über 90 °C, bevorzugt über 60 °C und besonders bevorzugt über 40 °C anwendet.

Ein Verfahren zur Herstellung von 3-[2-(Dimethylamino)methyl-(cyclohex-1-yl)]-phenol-Fumarat der Formel I in der kristallinen Form B, umfasst die Schritte
a) Lösen oder Suspendieren von 3-[2-(Dimethylamino)methyl-(cyclohex-1-yl)]-phenol in einem Lösungsmittel, oder Vorlegen von 3-[2-(Dimethylamino)methyl-(cyclohex-1-yl)]-phenol in Substanz,
b) Vermischen der Lösung, Suspension oder Substanz mit Fumarsäure, einer Suspension oder einer Lösung von Fumarsäure in einem Lösungsmittel, gegebenenfalls Abkühlen und Halten bei niedriger Temperatur bis zur vollständigen Bildung der kristallinen Form B, und
c) Isolieren der Verbindung der Formel I in der kristallinen Form B,
wobei in keiner Verfahrensstufe die Temperatur über 90 °C liegt und die Schritte a) und b) auch vertauscht sein können.

Die Temperatur beträgt in Verfahrensstufe b) beim Vermischen bevorzugt nicht mehr als 70 °C, bevorzugter nicht mehr als 60 °C und noch weiter bevorzugt nicht mehr als 40 °C. Abkühlen kann eine Temperatur bis etwa -20 °C, bevorzugter bis -10 °C und besonders bevorzugt bis 0 °C bedeuten. Die Temperatur liegt beim Lösen gemäss Verfahrensstufe a) im allgemeinen höher als beim Vermischen gemäss Verfahrensstufe b). Wenn beim Lösen eine Temperatur über 40 °C angewendet wird, wird die Lösung vor dem Vermischen auf 40 °C oder weniger abgekühlt, und gegebenenfalls einige Zeit bei dieser Temperatur gehalten.

Die freie Base und Fumarsäure können im molaren Verhältnis von 1:1 eingesetzt werden, oder Fumarsäure kann auch im Überschuss verwendet werden, zum Beispiel in einem molaren Verhältnis von bis zu 1,3, bevorzugt bis zu 1,1. Bei Verwendung eines Überschusses der freien Base werden keine HemiFumarate gebildet, selbst wenn man ein molares Verhältnis von Base zu Fumarsäure von 2:1 einstellt.

Die Menge von 3-[2-(Dimethylamino)methyl-(cyclohex-1-yl)]-phenol in der Lösung kann zum Beispiel 5 bis 70 Gew.-%, bevorzugt 10 bis 60 Gew.-%, bevorzugter 10 bis 50 Gew.-%, und besonders bevorzugt 15 bis 40 Gew.-% betragen, bezogen auf die Lösung. Die Lösung von 3-[2-(Dimethylamino)methyl-(cyclohex-1-yl)]-phenol kann erwärmt und anschliessend gegebenenfalls auf die Temperatur abgekühlt werden, die für das Vermischen mit Fumarsäure gewünscht ist.

Inerte (Kompatible) Lösungsmittel für 3-[2-(Dimethylamino)methyl-(cyclohex-1-yl)]-phenol und Fumarsäure sind zum Beispiel aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, t-Butylmethylether, Ethylenglykoldimethylether, Ethylenglykoldiethylether, Diethylenglykoldimethylether, Tetrahydrofuran, Dioxan), Ketone (Aceton, 2-Butanon, Methylisobutylketon), Carbonsäureester und Lactone (Essigsäureethyl- oder -methylester, Valerolacton), N-substituierte Lactame (N-Methylpyrrolidon), Carbonsäureamide (Dimethylacetamid, Dimethylformamid), acyclische Harnstoffe (Dimethylimidazolin), und Sulfoxide und Sulfone (Dimethylsulfoxid, Dimethylsulfon, Tetramethylensulfoxid, Tetramethylensulfon) und Alkohole (Methanol, Ethanol, 1- oder 2-Propanol, n-, i- und t-Butanol, 1-Pentanol, 1-Hexanol, 1-Heptanol, 1-Octanol, Ethylenglykolmonomethylether, Ethylenglykolmonoethylether, Diethylenglykolmonomethyl- oder monoethylether) und Wasser. Die Lösungsmittel können alleine oder in Mischung von wenigstens zwei Lösungsmitteln verwendet werden. Vorteilhaft verwendet man physiologisch unbedenkliche Lösungsmittel, die dem Fachmann geläufig sind.

Das Vermischen in der Verfahrensstufe b) kann mittels langsamer oder schneller Zugabe von einer Lösung zur anderen Lösung erfolgen. Eine oder beide Lösungen können erwärmt sein. Das Vermischen kann aber auch so vorgenommen werden, dass eine oder beide Lösungen sich bei Raumtemperatur befinden oder gekühlt sind, zum Beispiel bis auf -20 °C und bevorzugter bis auf -10 bis +10 °C, äußerst bevorzugt -5 bis +5 °C.. Nach dem Vermischen kann erwärmt und wieder gekühlt und es kann für einen bestimmten Zeitraum weitergerührt werden. Eine Kristallbildung kann auch durch Animpfen gefördert werden.

In der Regel bildet sich schon während des Mischens ein weisser Niederschlag, der kristallin und gut filtrierbar ist. Die Isolierung kann durch Dekantieren, Filtrieren oder Zentrifugieren erfolgen. Der kristalline Rückstand kann dann noch getrocknet werden, zum Beispiel mittels Erwärmen, Vakuumtrocknung oder Erwärmen im Vakuum, oder mittels eines gegebenenfalls erwärmten und inerten Gasstroms (Luft, Stickstoff, Edelgase). Die Verbindungen der Formel I werden in hohen Ausbeuten und hoher Reinheit erhalten. In der Regel sind keine oder nur wenige weitere Reinigungsschritte wie zum Beispiel Umkristallisationen erforderlich und das Produkt kann direkt zur Herstellung von pharmazeutischen Zusammensetzungen verwendet werden.

Ein weiterer Gegenstand der vorliegenden Erfindung ist ein Salz von Fumarsäure und 3-[2-(Dimethylamino)methyl-(cyclohex-1-yl)]-phenol der Formel I, erhältlich nach einem der vorstehend beschriebenen Verfahren.

Die Verbindungen der Formel I und das erfindungsgemässe Herstellungsverfahren bietet gegenüber entsprechenden Hydrochloriden erhebliche und teilweise unerwartete Vorteile. Da sich keine Hydrate und Solvate charaktisieren liessen, ist die Auswahl an einsetzbaren Lösungsmitteln breit und unkritisch. Die Stabilität an Luft und gegen Feuchtigkeit erlaubt ein offenes Handhaben ohne besondere Schutzmassnahmen Die spontane Salzbildung und Bildung von kristallinen Niederschlägen sowie deren gute Filtrierbarkeit erlauben Verfahren im industriellen Massstab.

Die Verbindungen der Formel I werden beim erfindungsgemässen Verfahren der Salzbildung als kristalliner Festkörper überwiegend in einer polymorphen Form erhalten, die nachfolgend als Form B bezeichnet wird. Amorphe Formen der Verbindungen der Formel I sind einfach zum Beispiel mittels Gefriertrocknung beziehungsweise schnellem Abkühlen von Lösungen erhältlich. Amorphe Verbindungen der Formel I sind nicht sehr stabil und neigen in Gegenwart von Feuchtigkeit zur Kristallisation. Bei Temperaturen von über 40 °C können sich Mischungen aus der kristallinen Form B und der bei höheren Temperaturen thermodynamisch stabileren, kristallinen Form A bilden. Die amorphe Form eignet sich besonders als Ausgangsmaterial zur gezielten Herstellung von kristallinen Formen.

Es wurde gefunden, dass die Verbindungen der Formel I als kristalline Festkörper polymorphe Formen bilden, die gezielt aus 3-[2-(Dimethylamino)methyl-(cyclohex-1-yl)]-phenol-Fumarat herstellbar sind und auf Grund ihrer Stabilität besonders als Wirkstoff zur Formulierung von pharmazeutischen Zusammensetzungen geeignet sind. Es ist bekannt [siehe zum Beispiel Z. Jane Li et al. in J. Pharm. Sci., Vol. 88(3), Seiten 337 bis 346 (1999)], dass Enantiomere identische Röntgen-Diffraktogramme und Raman-Spektren ergeben und somit gleiche polymorphe Formen bilden. Im Rahmen der Erfindung werden somit polymorphe Formen von allen Enantiomeren umfasst.

Ein weiterer Gegenstand der Erfindung ist eine kristalline Form B von 3-[2-(Dimethylamino)methyl-(cyclohex-1-yl)]-phenol-fumarat der Formel I, die ein charakteristisches Röntgen-Beugungsbild mit den nachstehenden ausgeprägten Reflexen aufweist.

| | **2Theta** | **rel. Intensität** |
|---|---|---|
| | 9,52 | 100 |
| | 10,40 | 12 |
| | 12,61 | 21 |
| | 13,28 | 34 |
| | 16,55 | 72 |
| | 17,05 | 24 |
| | 17,23 | 8 |
| | 19,14 | 80 |
| | 19,60 | 33 |
| | 20,86 | 94 |
| | 21,27 | 33 |
| | 21,95 | 11 |
| | 22,50 | 27 |
| | 23,51 | 27 |
| | 23,87 | 7 |
| | 24,96 | 14 |
| | 25,42 | 32 |
| | 25,72 | 14 |
| | 26,76 | 35 |
| | 27,07 | 12 |
| | 28,03 | 5 |
| | 28,87 | 7 |
| | 29,26 | 18 |
| | 30,04 | 4 |
| | 30,36 | 6 |
| | 30,74 | 5 |
| | 31,63 | 13 |
| | 32,76 | 5 |
| | 33,47 | 5 |
| | 34,86 | 10 |
| | 35,16 | 5 |
| | 36,15 | 6 |
| | 37,82 | 5 |
| | 38,88 | 6 |
| | 39,76 | 9 |
| | 41,57 | 7 |
| | 42,44 | 5 |
| | 44,44 | 5 |
| | 45,83 | 5 |
| | 46,67 | 4 |

In der vorstehenden Tabelle sind die Peaklagen (in 2 Theta) sowie die relativen Intensitäten der Peaks angegeben. Der intensivste Peak ist dabei auf eine relative Intensität von 100 normiert.

Die kristalline Form 13 von 3-[2-(Dimethylamino)methyl-(cyclohex-1-yl)]-phenol-Fumarat der Formel I, weist ein charakteristisches Röntgen-Beugungsbild im Bereich von 2° bis 35° 2θ mit ausgeprägten charakteristischen Linien auf, ausgedrückt in d-Werten (Å): 9,3 (vs), 7,0 (m), 6.7 (s), 5,37 (s), 5,21 (s), 4,64 (s), 4,52 (s), 4,28 (vs), 4,23 (s), 4,19 (s), 3,94 (m) 3,78 (m), 3,52 (m), 3,49 (m), 3,33 (s), 3,30 (m), 3,06 (s), 2,83 (m);

Zuvor und nachfolgend bedeuten die Abkürzungen in Klammern: (vs) = sehr starke Intensität, (s) = starke Intensität, (m) = mittlere Intensität, (w) = schwache Intensität, und (vw) = sehr schwache Intensität. Die Abkürzung, "sh" in den Tabellen der Ramanspektren bedeutet Schulter.

Die kristalline Form 13 von 3-[2-(Dimethylamino)methyl-(cyclohex-1-yl)]-phenol-Fumarat der Formel I, weist ein charakteristisches Raman Spektrum mit charakteristischen Linien auf, ausgedrückt in Wellenzahlen (cm⁻¹):

| Position (cm-1) | Intensität |
|---|---|
| 3070 | m |
| 3051 | m |
| 3031 | m |
| 3016 | m |
| 2991 | w |
| 2964 | m |
| 2930 | s |
| 2918 | s |
| 2898 | sh, m |
| 2878 | m |
| 2859 | s |
| 2813 | vw |
| 1683 | m |
| 1601 | m |
| 1472 | w |
| 1459 | m |
| 1444 | m |
| 1388 | m |
| 1350 | w |
| 1331 | w |
| 1323 | w |
| 1307 | w |
| 1295 | m |
| 1267 | w |
| 1246 | w |
| 1240 | w |
| 1236 | w |
| 1211 | w |
| 1177 | w |
| 1162 | w |
| 1121 | vw |
| 1106 | w |
| 1083 | w |
| 1074 | w |
| 1058 | w |
| 1048 | w |
| 1000 | vs |
| 972 | w |
| 957 | m |
| 930 | w |
| 905 | w |
| 857 | w |
| 841 | m |
| 821 | w |
| 797 | w |
| 763 | vw |
| 753 | m |
| 704 | vw |
| 634 | w |
| 620 | vw |
| 610 | vw |
| 571 | vw |
| 535 | m |
| 512 | vw |
| 469 | vw |
| 453 | vw |
| 412 | w |
| 355 | w |
| 347 | w |
| 325 | w |
| 279 | m |
| 246 | s |
| 239 | sh, m |
| 171 | m |
| 109 | vs |
| 89 | vs |
| 78 | vs |

Die kristalline Form B von 3-[2-(Dimethylamino)methyl-(cyclohex-1-yl)]-phenol-fumarat der Formel I weist die charakteristische Banden im Raman Spektrum, ausgedrückt in Wellenzahlen (cm-1), bei 171 (m) auf.

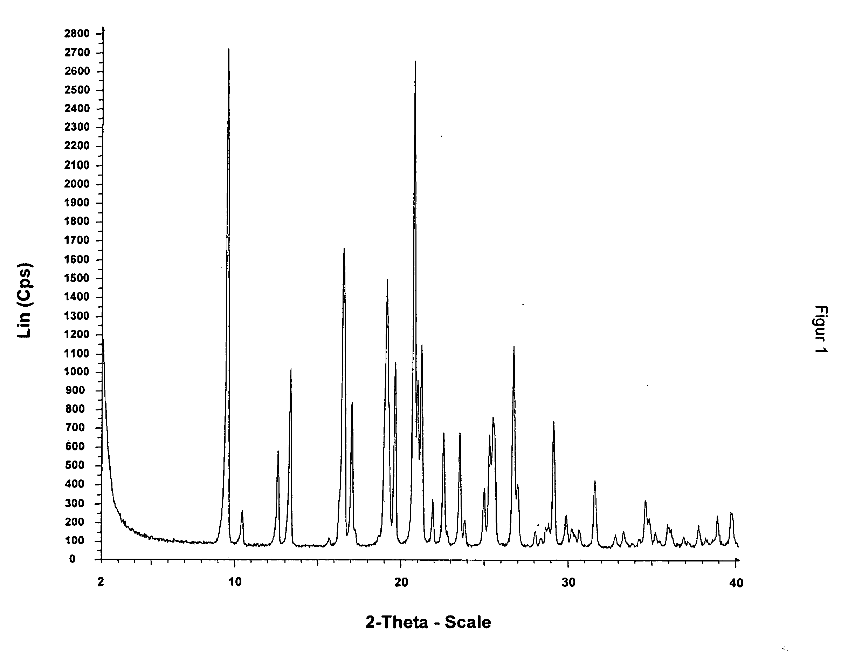

Erfindungsgegenstand ist auch eine kristalline Form B von 3-[2-(Dimethylamino)methyl-(cyclohex-1-yl)]-phenol-Fumarat der Formel I, die ein Röntgenbeugungsbild wie in Figur 1 dargestellt aufweist.

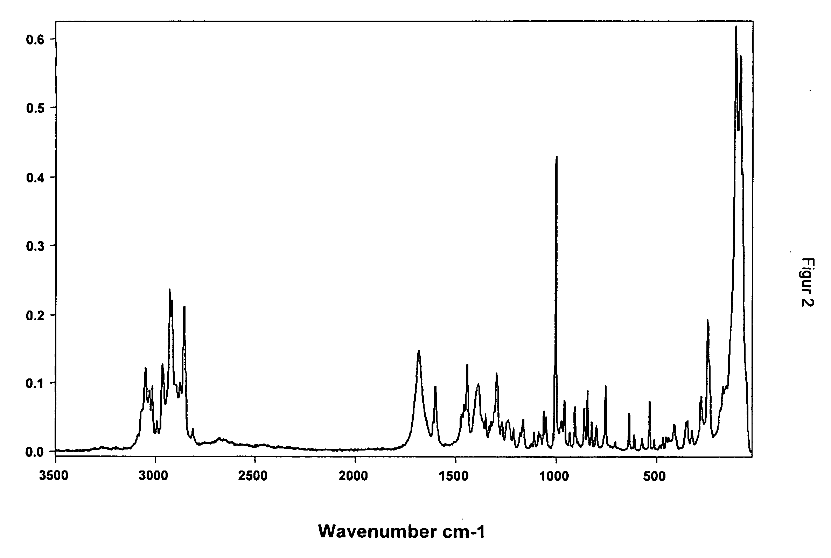

Erfindungsgegenstand ist auch eine kristalline Form B von 3-[2-(Dimethylamino)methyl-(cyclohex-1-yl)]-phenol-Fumarat der Formel I, die ein Ramanspektrum wie in Figur 2 dargestellt aufweist.

Die kristalline Form B ist die thermodynamisch stabilste Form bei niedriger Temperatur bis zum Beispiel etwa 40 °C und weist zusätzlich eine ausgezeichnete chemische und physikalische Stabilität auf. Polymorph B ist sogar gegen den Einfluss von Luftfeuchtigkeit bei hohen relativen Luftfeuchtigkeiten bis zu 90% unempfindlich und stabil, selbst über einen längeren Zeitraum. Es wird keine Wasseraufnahme, keine Bildung von Hydraten und keine Umwandlung in andere kristalline Formen bei Normalbedingungen beobachtet. Polymorph B zeigt auch keine Phasentransformationen in Luft und Gegenwart von Feuchtigkeit. Polymorph B verändert sich aber unter erhöhtem Druck oder beim Mahlen, und man beobachtet eine Umwandlung in die kristalline Form A unter Einwirkung von höherem Druck. Polymorph B ist nicht hygroskopisch und absorbiert nur geringe Mengen

Oberflächenwasser. Polymorph B bildet unter diesen Bedingungen auch keine Solvate und im Kontakt mit Lösungsmitteln wird keine Umwandlung beobachtet. Die Löslichkeit in polaren Lösungsmitteln ist sehr gut. Der Schmelzpunkt beträgt etwa 176 °C und die Schmelzenthalpie beträgt etwa 113 J/g, bestimmt mittels DSC bei einer Aufheizrate von 10 °C/Minute. Die Umwandlungstemperatur in die kristalline Form A liegt bei etwa über 40 °C. Polymorph B kann als festes Pulver mit gewünschten mittleren Partikelgrössen hergestellt werden, die in der Regel im Bereich von 1 µm bis etwa 500 µm liegen. Die kristalline Form B eignet sich wegen seiner Eigenschaften am Besten für die Herstellung pharmazeutischer Formulierungen.

Die Verbindung der Formel I bildet eine weitere zur kristallinen Form B sehr ähnliche kristalline Form B', die reproduzierbar hergestellt werden kann und sich zum Beispiel im Röntgenbeugungsbild im wesentlichen durch die Intensität der Peaks und der Lage bei 19 bis 19,4° 2θ unterscheiden. Die Eigenschaften entsprechen im wesentlichen der Form B.

Die Verbindung der Formel I bildet eine weitere, bei höheren Temperaturen thermodynamisch stabile kristalline Form A, die bei Normalbedingungen an Luft und unter Ausschluss von Luftfeuchtigkeit ebenfalls stabil ist. Die kristalline Form A ist auch so handhabbar, dass sie für die Herstellung pharmazeutischer Zusammensetzungen eingesetzt werden kann.

Ein weiterer Gegenstand der Erfindung ist eine kristalline Form A von 3-[2-(Dimethylamino)methyl-(cyclohex-1-yl)]-phenol-fumarat der Formel I, die ein charakteristisches Röntgen-Beugungsbild mit den nachstehenden ausgeprägten Reflexen aufweist.

| **2Theta** | **rel. Intensität** |
|---|---|
| 9,51 | 100 |
| 10,43 | 10 |
| 12,58 | 21 |
| 13,30 | 37 |
| 15,66 | 4 |
| 16,51 | 61 |
| 17,03 | 31 |
| 17,25 | 6 |
| 19,12 | 53 |
| 19,26 | 30 |
| 19,63 | 39 |
| 20,77 | 96 |
| 20,99 | 35 |
| 21,22 | 42 |
| 21,92 | 12 |
| 22,57 | 25 |
| 22,77 | 6 |
| 23,55 | 25 |
| 23,86 | 8 |
| 25,00 | 13 |
| 25,33 | 24 |
| 25,54 | 27 |
| 25,60 | 27 |
| 26,76 | 42 |
| 26,99 | 15 |
| 28,07 | 6 |
| 28,42 | 4 |
| 28,68 | 6 |
| 28,88 | 7 |
| 29,17 | 27 |
| 29,91 | 9 |
| 30,25 | 6 |
| 30,68 | 6 |
| 31,61 | 15 |
| 32,85 | 5 |
| 33,34 | 5 |
| 34,65 | 12 |
| 34,87 | 7 |
| 35,24 | 5 |
| 35,97 | 7 |
| 37,82 | 7 |
| 38,94 | 8 |
| 39,76 | 9 |
| 40,72 | 4 |
| 41,55 | 10 |
| 42,35 | 7 |
| 43,35 | 5 |
| 44,50 | 6 |
| 45,84 | 6 |
| 46,64 | 4 |

In der vorstehenden Tabelle sind die Peaklagen (in 2 Theta) sowie die relativen Intensitäten der Peaks angegeben. Der intensivste Peak ist dabei auf eine relative Intensität von 100 normiert.

Die kristalline Form A von 3-[2-(Dimethylamino)methyl-(cyclohex-1-yl)]-phenol-Fumarat der Formel I weist ein charakteristisches Röntgen-Beugungsbild im Bereich von 2° bis 35° 2θ mit ausgeprägten charakteristischen Linien auf, ausgedrückt in d-Werten (Å): 9,3 (vs), 7,0 (m), 6,7 (s), 5,36 (vs), 5,20 (m) 4,64 (vs), 4,53 (s), 4,26 (vs), 4,18 (s), 3,95 (m), 3,78 (m), 3,57 (m), 3,50 (s), 3,46 (m), 3,33 (s), 3,05 (m), 2,83 (m);

Die kristalline Form A von 3-[2-(Dimethylamino)methyl-(cyclohex-1-yl)]-phenol-Fumarat der Formel I, weist ein charakteristisches Raman Spektrum mit charakteristischen Linien auf, ausgedrückt in Wellenzahlen (cm-1):

| Position (cm-1) | Intensität |
|---|---|
| 3070 | m |
| 3050 | m |
| 3031 | m |
| 3017 | m |
| 2991 | w |
| 2964 | m |
| 2931 | s |
| 2919 | s |
| 2898 | sh, m |
| 2872 | sh, m |
| 2859 | s |
| 2812 | vw |
| 1705 | sh, m |
| 1685 | m |
| 1644 | w |
| 1602 | m |
| 1470 | w |
| 1459 | m |
| 1445 | m |
| 1386 | m |
| 1351 | w |
| 1331 | w |
| 1323 | w |
| 1316 | w |
| 1306 | w |
| 1293 | m |
| 1269 | w |
| 1247 | w |
| 1240 | w |
| 1236 | w |
| 1211 | w |
| 1176 | w |
| 1163 | w |
| 1121 | vw |
| 1107 | w |
| 1083 | w |
| 1075 | w |
| 1058 | w |
| 1048 | w |
| 1000 | vs |
| 972 | w |
| 957 | m |
| 931 | w |
| 904 | w |
| 857 | w |
| 842 | m |
| 821 | w |
| 797 | w |
| 763 | w |
| 753 | m |
| 706 | vw |
| 634 | w |
| 620 | vw |
| 610 | vw |
| 571 | vw |
| 535 | m |
| 512 | vw |
| 468 | vw |
| 453 | vw |
| 413 | w |
| 355 | w |
| 347 | w |
| 326 | w |
| 280 | m |
| 246 | s |
| 239 | sh, m |
| 108 | vs |
| 89 | vs |
| 78 | vs |

Die kristalline Form A von 3-[2-(Dimethylamino)methyl-(cyclohex-1-yl)]-phenol-fumarat der Formel I weist eine oder beide der charakteristischen Banden im Raman Spektrum, ausgedrückt in Wellenzahlen (cm-1), bei 1644 (w) und 1705 (sh, m) auf,

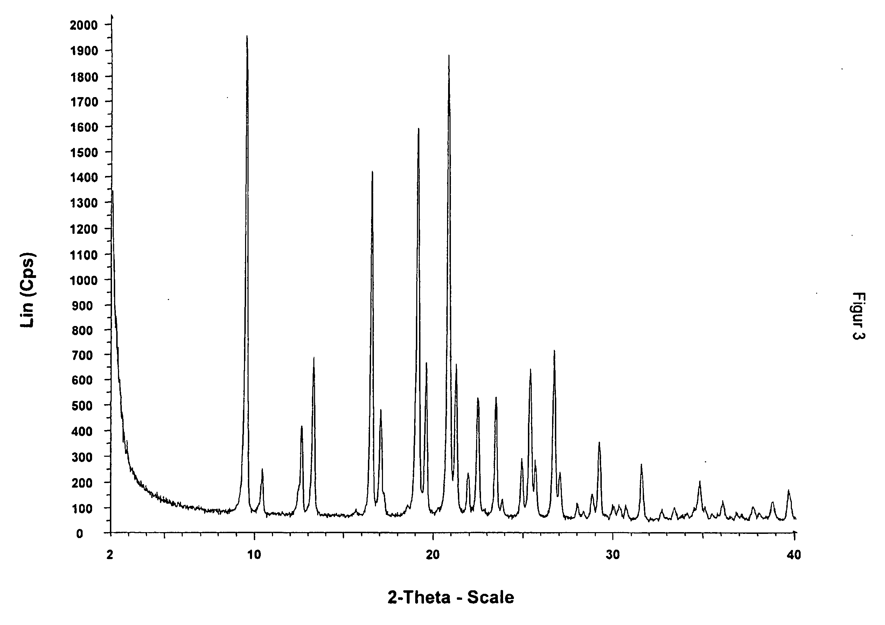

Erfindungsgegenstand ist auch eine kristalline Form A von 3-[2-(Dimethylamino)methyl-(cyclohex-1-yl)]-phenol-Fumarat der Formel I, die ein Röntgenbeugungsbild wie in Figur 3 dargestellt aufweist.

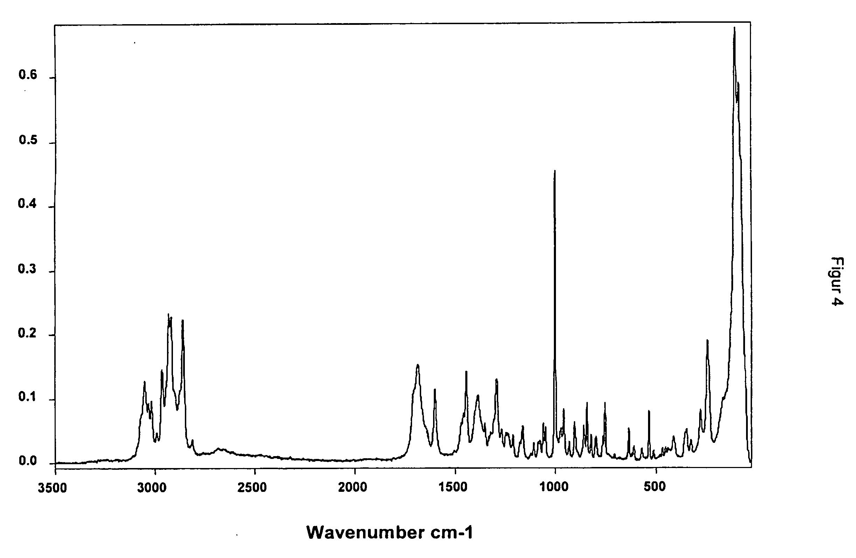

Erfindungsgegenstand ist weiter eine kristalline Form A von 3-[2-(Dimethylamino)methyl-(cyclohex-1-yl)]-phenol-Fumarat der Formel I, die ein Ramanspektrum wie in Figur 4 dargestellt aufweist.

Die kristalline Form A ist die thermodynamisch stabilste Form bei höherer Temperatur, zum Beispiel etwa mindestens 40 °C oder mehr. Polymorph A weist zusätzlich eine ausgezeichnete chemische Stabilität bei erhöhten Temperaturen auch in Gegenwart von Luftfeuchtigkeit auf. Die physikalische Stabilität ist geringer, denn bei erhöhtem Druck, und schon bei Raumtemperaturen mit oder ohne Feuchtigkeitseinfluss tritt eine Umwandlung in Polymorph B ein. Es wird aber keine Wasseraufnahme und keine Bildung von Hydraten beobachtet. Polymorph A bildet auch keine Solvate. Im Kontakt mit Lösungsmitteln kann eine Umwandlung in die kristalline Form B eintreten. Die Löslichkeit in polaren Lösungsmitteln ist sehr gut und vergleichbar mit der Löslichkeit von Polymorph B. Der Schmelzpunkt beträgt etwa 175,5 °C und die Schmelzenthalpie beträgt etwa 111 J/g, bestimmt mittels DSC bei einer Aufheizrate von 10 °C/Minute. Polymorph A kann als festes Pulver mit gewünschten mittleren Partikelgrössen hergestellt werden, die in der Regel im Bereich von 1 µm bis etwa 500 µm liegen. Eine Lagerung der kristallinen Form A unter trockenem Schutzgas (zum Beispiel Stickstoff) ist empfehlenswert.

Die polymorphe Form A kann sich in die kristalline Form B (B') umwandeln und umgekehrt. Die Formen A und B bilden ein enantiotropes System mit einem Übergangspunkt bzw. -bereich von etwa 40 bis 60°C. Gegenstand der Erfindung sind daher auch Mischungen der kristallinen Formen A und B in an sich beliebigen Mengenverhältnissen. Die Kristallgitter der Formen A und B sind sich sehr ähnlich, was sich zum Beispiel darin zeigt, dass die Raman-Spektren und Röntgenbeugungsbilder nur geringe Unterschiede aufweisen. Neben unterschiedlicher Intensitäten gleichlagiger Peaks weisen Form A zwei Peaks im Bereich von 21° 2θ auf und Form B drei Peaks.

Bezüglich der angegebenen Schmelzpunkte und Schmelzenthalpien wird angemerkt, dass bei der Differential Scanning Kaolrimetrie (DSC) sehr ähnliche Werte für die verschiedenen Kristallformen gefunden werden. Bei einer temperaturabhängigen Aufnahme von Röntgenbeugungsbildern, ausgehend von Form B, wurde gefunden, dass bei steigender Temperatur zunächst eine vollständige Umwandlung in die kristalline Form A erfolgt (etwa 50°C) und dann bei etwa 150°C eine Umwandlung in eine neue, nur bei hohen Temperaturen und bis zum Schmelzpunkt stabile Form C beobachtet wird. Es kann daher nicht ausgeschlossen werden, dass die Werte für Schmelzpunkt und Schmelzenthalpie der Form C zuzuordnen sind.

Die polymorphen Formen können nach an sich bekannten Kristallisationsverfahren aus dem Salz 3-[2-(Dimethylamino)methyl-(cyclohex-1-yl)]-phenol-Fumarat hergestellt werden, zum Beispiel Rühren von Suspensionen (Einstellung von Phasengleichgewichten), Fällung, Umkristallisation, oder Verdampfung von Lösungsmitteln. Es können verdünnte, gesättigte oder übersättigte Lösungen verwendet werden, mit oder ohne Animpfen mit einem Kristallkeimbildner. Die Temperaturen zur Bildung von Lösungen können bis zu 100 °C betragen. Die Kristallisation kann durch Kühlen auf etwa -100 °C bis 30 °C, und bevorzugt -30 °C bis 20 °C initiert werden, wobei ein Abkühlen kontinuierlich oder stufenweise erfolgen kann. Zur Herstellung von Lösungen oder Suspensionen können amorphe oder kristalline Startmaterialien verwendet werden, um hohe Konzentrationen in Lösungen zu erzielen und andere kristalline Formen zu erhalten.

Ein Verfahren zur Herstellung der kristallinen Form B von 3-[2-(Dimethylamino)methyl-(cyclohex-1-yl)]-phenol-Fumarat, ist dadurch gekennzeichnet dass man
a) eine pulverförmige, feste, amorphe Form von 3-[2-(Dimethylamino)methyl-(cyclohex-1-yl)]-phenol-Fumarat mit einem Wasserdampf enthaltenden inerten Gas bis zur vollständigen Bildung der kristallinen Form B behandelt; oder
b) eine Suspension der amorphen Form von 3-[2-(Dimethylamino)methyl-(cyclohex-1-yl)]-phenol-Fumarat in einem Lösungsmittel als Träger herstellt und bis zur vollständigen Bildung der kristallinen Form B rührt; oder
c) 3-[2-(Dimethylamino)methyl-(cyclohex-1-yl)]-phenol-Fumarat in einem Lösungsmittel löst, anschliessend ausfällt und gegebenenfalls bis zur vollständigen Bildung der kristallinen Form B rührt;
wobei die Temperatur in den Verfahrensstufen a), b) und c) höchstens 90 °C, bevorzugt höchstens 60 °C und besonders bevorzugt höchstens 40°C beträgt.

Die Temperatur in den Verfahrensschritten a) und b) beträgt bevorzugt höchstens 40 °C und das Verfahren wird besonders bevorzugt bei Raumtemperatur durchgeführt. Inerte Gase sind zum Beispiel Luft, Stickstoff und Edelgase, wobei Luft schon aus wirtschaftlichen Gründen besonders bevorzugt ist. Die relative Feuchtigkeit der Gase kann zum Beispiel 40 bis 90% und bevorzugt 60 bis 90 % betragen. Die Behandlungszeit in Verfahrensstufe a) hängt im wesentlichen von der Partikelgrösse und der relativen Feuchtigkeit ab und kann zum Beispiel 5 bis 100 Stunden betragen. In Verfahrensstufe a) kann auch das Polymorph B neben der Form A gebildet werden, wenn die relative Luftfeuchtigkeit zu niedrig und/oder die Behandlungszeit zu kurz ist. Nach der Isolierung kann der kristalline Rückstand in üblicher Weise getrocknet werden, wobei man zweckmässig Temperaturen über 40 °C vermeidet.

Der Verfahrensschritt b) wird vorzugsweise bei etwa -20 ° bis 40 °C und besonders bevorzugt bei -5 °C bis 25 °C (etwa Raumtemperatur) durchgeführt. In Frage kommende Lösungsmittel sind zuvor genannt worden. Die Behandlungszeit in Verfahrensstufe b) kann 5 bis 100 Stunden betragen. Nach der Isolierung kann das verwendete Lösungsmittel beziehungsweise Lösungsmittelgemisch in üblicher Weise mittels bekannter Trocknungsverfahren entfernt werden.

Im Verfahrensschritt c) kann die kristalline Form A, B oder die amorphe Form von 3-[2-(Dimethylamino)methyl-(cyclohex-1-yl)]-phenol-Fumarat zur Herstellung von Lösungen verwendet werden. Die Konzentration an 3-[2-(Dimethylamino)methyl-(cyclohex-1-yl)]-phenol-Fumarat in der Lösung hängt von der gewählten Temperatur und vom Lösungsmittel ab. Die gelöste Menge kann zum Beispiel von 0,5 bis 50, bevorzugt 0,5 bis 30, bevorzugter 0,5 bis 20 und besonders bevorzugt 1 bis 15 Gewichtsprozent betragen, bezogen auf das Lösungsmittel. Die Temperatur zum Lösen kann bis zu 70 °C und bevorzugt bis zu 60 °C und bevorzugter bis zu 40°C betragen. Die Lösung kann auch bei Raumtemperatur hergestellt werden, wenn man Lösungsmittel mit hohem Lösungsvermögen einsetzt, zum Beispiel Wasser, Aceton, Dimethylformamid, Dimethylsulfoxid, Methanol, Ethanol, N-Methylpyrrolidon und Propylenglykol. Die Fällung kann mittels Abkühlen, teilweiser oder vollständiger Entfernung des Lösungsmittels, Zugabe eines Fällungsmittels (Nichtlösungsmittel, zum Beispiel Heptan oder Methyl-t-butylether) oder einer Kombination dieser Massnahmen erfolgen. Abkühlen kann langsames Abkühlen oder auch Abschrecken auf Temperaturen bis -20 °C und bevorzugt bis 0°C bedeuten. Das Lösungsmittel kann durch Erwärmen, im Gasstrom, Anlegen von Vakuum oder einer Kombination dieser Massnahmen erfolgen. Erwärmen zum Entfernen von Lösungsmittel bedeutet in der Verfahrensstufe c) eine Temperatur von höchstens 40 °C und vorzugsweise höchstens 30 °C. Bei den Fällungsverfahren gemäss Verfahrensschritt c) werden bevorzugt die erhaltenen Suspensionen für eine längere Zeit bei Temperaturen von - 20 °C und bevorzugt 0°C bis 10 °C nachgerührt, um möglicherweise gebildete kristalline Form A in die polymorphen Form B umzuwandeln.

Die Herstellung der polymorphen Form B ist relativ unkritisch. Für die Herstellung können auch Verdampfungsverfahren eingesetzt werden. Bei diesem Verfahren ist die Wahl der Lösungsmittel und besonders die Verdampfungstemperatur zu beachten. Bei Temperaturen um Raumtemperatur wird überwiegend die polymorphe Form B gebildet. Bei Temperaturen von über 40 °C und vorzugsweise ab 50°C wird überwiegend die polymorphe Form A gebildet. Ferner wird die Bildung von Mischungen der kristallinen Formen A und B auch bei Raumtemperatur beobachtet.

Für die gezielte Herstellung der kristallinen Form A können grundsätzlich die gleichen Verfahren wie für die Herstellung der kristallinen Form B verwendet werden, wobei die Temperatur in den einzelnen Verfahrensschritten über 40 °C gehalten wird. Die Temperatur kann zum Beispiel 50 °C bis 120 °C und bevorzugt 50 °C bis 100 °C betragen. Das Halten bei tieferen Temperaturen nach der Abkühlung beträgt vorzugsweise nicht mehr als 2 und vorteilhafter 1 Tag.

Ein Verfahren zur Herstellung der kristallinen Form A von 3-[2-(Dimethylamino)methyl-(cyclohex-1-yl)]-phenol-Fumarat der Formel I, ist dadurch gekennzeichnet, dass man
a) eine pulverförmige, feste, amorphe Form von 3-[2-(Dimethylamino)methyl-(cyclohex-1-yl)]-phenol-Fumarat mit einem Wasserdampf enthaltenden inerten Gas bis zur vollständigen Bildung der kristallinen Form A behandelt; oder
b) eine Suspension der amorphen Form von 3-[2-(Dimethylamino)methyl-(cyclohex-1-yl)]-phenol-Fumarat in einem Lösungsmittel als Träger herstellt und bis zur vollständigen Bildung der kristallinen Form A rührt; oder
c) 3-[2-(Dimethylamino)methyl-(cyclohex-1-yl)]-phenol-Fumarat in einem Lösungsmittel löst, anschliessend ausfällt und gegebenenfalls bis zur vollständigen Bildung der kristallinen Form A rührt;
wobei in den Verfahrensstufen a), b) und c) die Temperatur mehr als 90 °C, bevorzugt mehr als 60 °C und besonders bevorzugt mehr als 40 °C beträgt und die kristalline Form nach dem Abkühlen isoliert wird.

3-[2-(Dimethylamino)methyl-(cyclohex-1-yl)]-phenol-Fumarat ist auf Grund seines günstigen Gesamteigenschaftsprofils hervorragend als Wirkstoff für pharmazeutische Zusammensetzungen und ganz besonders für schmerzstillende Medikamente geeignet. Demgemäss ist auch ein Erfindungsgegenstand die Verwendung von 3-[2-(Dimethylamino)methyl-(cyclohex-1-yl)]-phenol-Fumarat der Formel I als Wirkstoff in Arzneimitteln, bevorzugt als Wirkstoff in Schmerzmitteln. Bevorzugt sind auch hier, wie in der ganzen Anmeldung, Diastereomere oder Gemische von enantiomeren Diastereomeren mit Transkonfiguration des Phenylringes und der Dimethylaminomethylgruppe (1 R,2R- beziehungsweise 1S,2S-Konfiguration), wobei das Enantiomer mit der absoluten Konfiguration (1R,2R) ganz besonders bevorzugt ist.

Ein weiterer Gegenstand der Erfindung ist eine pharmazeutische Zusammensetzung, enthaltend eine wirksame Menge 3-[2-(Dimethylamino)methyl-(cyclohex-1-yl)]-phenol-Fumarat der Formel I und einen pharmazeutischen Träger oder ein pharmazeutisches Verdünnungsmittel.

In der Zusammensetzung kann die Verbindung der Formel I als kristalline Form A, kristalline Form B oder einer Mischung der Formen A und B vorliegen. Vorzugsweise ist die kristalline Form B enthalten.

Die Menge an Verbindungen der Formel I hängt im wesentlichen vom Formulierungstyp und von der gewünschten Dosierung während des Zeitraums der Verabreichung ab. Die an den Patienten zu verabreichende Menge der jeweiligen erfindungsgemäßen Salze kann variieren und ist beispielsweise abhängig vom Gewicht oder Alter des Patienten sowie von der Applikationsart, der Indikation und dem Schweregrad der Erkrankung, Üblicherweise werden 0,005 bis 5000 mg/kg, vorzugsweise 0,05 bis 500 mg/kg Körpergewicht des Patienten wenigstens einer solchen Verbindung appliziert.

Bei oralen Formulierungen kann es sich um feste Formulierungen handeln, zum Beispiel Tabletten, Kapseln, Pillen und Pastillen. Bei oralen Formulierungen kann es sich auch um flüssige Formulierungen handeln, zum Beispiel Lösungen, Suspensionen, Sirupe oder Elexire. Flüssige und feste Formulierungen umfassen auch die Einarbeitung der Verbindungen der Formel I in feste oder flüssige Nahrungsmittel. Ferner umfassen Flüssigkeiten noch Lösungen für parenterale Applikationen wie zum Beispiel Infusion oder Injektion.

Die Verbindungen der Formel I und die kristallinen Formen können direkt als Pulver (mikronisierte Teilchen), Granulate, Suspensionen oder Lösungen verwendet werden, oder sie können mit anderen pharmazeutisch annehmbaren Zutaten und Komponenten vermischt und dann pulverisiert werden, um die Pulver dann in Kapseln aus Hart- oder Weichgelatine zu füllen, Tabletten, Pillen oder Pastillen zu pressen, oder um die Pulver in einem Träger zu suspendieren oder zu lösen zur Herstellung von Suspensionen, Sirupen oder Elexieren. Tabletten, Pillen oder Pastillen können nach dem Pressen mit einem Überzug versehen werden.

Pharmazeutisch annehmbare Zutaten und Komponenten für die verschiedenen Formulierungstypen sind an sich bekannt. Es kann sich zum Beispiel um Bindemittel wie synthetische oder natürliche Polymere, Arzneiträger, Gleitmittel, Tenside, Süssmittel und Aromastoffe, Beschichtungsmittel, Konservierungsmittel, Farbstoffe, Verdickungsmittel, Hilfsmittel, antimikrobielle Mittel und Träger für die verschiedenen Formulierungstypen handeln.

Beispiele für Bindemittel sind Gummi Arabicum, Gummi Tragakant, Akaziengummi und biologisch abbaubare Polymere wie Homo- oder Copolyester von Dicarbonsäuren, Alkylendiolen, Polyalkylenglykolen und/oder aliphatischen Hydroxycarbonsäuren; Homo- oder Copolyamide von Dicarbonsäuren, Alkylendiaminen und/oder aliphatischen Aminocarbonsäuren; entsprechende Polyester-polyamid-copolymere, Polyanhydride, Polyorthoester, Polyphosphazene und Polycarbonate. Die biologisch abbaubaren Polymere können linear, verzeigt oder vernetzt sein. Spezifische Beispiele sind Polyglykolsäure, Polymilchsäure und Poly-d,l-milch-/ glykolsäure. Andere Beispiele für Polymere sind wasserlösliche Polymere wie zum Beispiel Polyoxaalkylene (Polyoxaethylen, Polyoxapropylen und Mischpolymere davon), Polyacrylamide und hydroxylalkylierte Polyacrylamide, PolyFumarsäure und Ester oder Amide davon, Polyacrylsäure und Ester oder Amide davon, Polyvinylalkohol und Ester oder Ether davon, Polyvinylimidazol, Polyvinylpyrrolidon und natürliche Polymere wie zum Beispiel Chitosan.

Beispiele für Arzneiträger sind Phosphate wie Dicalciumphosphat.

Beispiele für Gleitmittel sind natürliche oder synthetische Öle, Fette, Wachse oder Fettsäuresalze wie Magnesiumstearat.

Tenside (oberflächenaktive Mittel) können anionisch, kationisch, amphoter oder neutral sein. Beispiele für Tenside sind Lecithin, Phospholipide, Octylsulfat, Decylsulfat, Dodecylsulfat, Tetradecylsulfat, Hexadecylsulfat und Octadecylsulfat, Natriumoleat oder Natriumcaprat, 1-Acylaminoethan-2-sulfonsäuren wie 1-Octanoylaminoethan-2-sulfonsäure, 1-Decanoylaminoethan-2-sulfonsäure, 1-Dodecanoylaminoethan-2-sulfonsäure, 1-Tetradecanoylaminoethan-2-sulfonsäure, 1-Hexadecanoylaminoethan-2-sulfonsäure, und 1-Octadecanoylaminoethan-2-sulfonsäure, Gallensäuren, Salze und Derivate davon, wie zum Beispiel Cholsäure, Deoxycholsäure, Taurocholsäure, Taurodeoxycholsäure und Natriumglycocholate, Natriumcaprat, Natriumlaurat, Natriumoleat, Natriumlaurylsulfat, Natriumcetylsulfat, sulfatiertes Castoröl, Natriumdioctylsulfosuccinat, Cocamidopropylbetain und Laurylbetain, Fettalkohole, Cholesterole, Glycerinmono- oder -distearat, Glycerinmono- oder -dioleat, Glycerinmono- oder -dipalmitat, und Polyoxyethylenstearat.

Beispiele für Süssmittel sind Sucrose, Fructose, Lactose oder Aspartam.

Beispiele für Aromastoffe sind Pfefferminz, Öl von Wintergrün oder Fruchtaroma wie Kirschen- oder Orangenaroma.

Beispiele für Beschichtungsmittel sind Gelatine, Wachse, Schellack, Zucker oder biologisch abbaubare Polymere.

Beispiele für Konservierungsmittel sind Methyl- oder Propylparaben, Sorbinsäure, Chlorbutanol und Phenol.

Beispiele für Hilfsmittel sind Duftstoffe.

Beispiele für Verdickungsmittel sind synthetische Polymere, Fettsäuren, Fettsäuresalze, Fettsäureester und Fettalkohole.

Beispiele für flüssige Träger sind Wasser, Alkohole (Ethanol, Glycerol, Propyleneglykol, flüssige Polyethylenglykole, Polytriazine and Öle. Beispiele für feste Träger sind Talk, Tonerden, mikrokristalline Cellulose, Siliziumdioxid, Aluminiumoxid und ähnliche Feststoffe.

Die erfindungsgemässe Zusammensetzung kann auch isotonische Mittel wie zum Beispiel Zucker, physiologische Puffer und Natriumchlorid enthalten.

Die erfindungsgemässe Zusammensetzung kann auch als Brausetablette oder Brausepulver formuliert sein, die sich in wässriger Umgebung zersetzt unter Zubereitung von Trinklösungen oder -suspensionen.

Ein Sirup oder Elexier kann die Verbindung der Formel I, einen Zucker wie Sucrose oder Fructose als Süssmittel, ein Konservierungsmittel (wie Methylparaben), einen Farbstoff und ein Geschmacksmittel (wie Aromastoffe) enthalten.

Bei der erfindungsgemässen Zusammensetzung kann es sich auch um Formulierungen mit verzögerter und kontrollierter Wirkstoffabgabe im Kontakt mit Körperflüssigkeiten des gastrointestinalen Traktes handeln, um einen im wesentlichen konstanten und effektiven Level des Wirkstoffs im Blutplasma zu erzielen. Die Verbindungen der Formel I können für diesen Zweck in eine Polymermatrix eines biologisch abbaubaren Polymers, eines wasserlöslichen Polymers oder beiden Polymeren eingebettet werden, gegebenenfalls zusammen mit einem geeigneten Tensid. Einbetten kann in diesem Zusammenhang die Einarbeitung von Mikropartikeln in die Polymermatrix bedeuten. Formulierungen mit verzögerter und kontrollierter Wirkstoffabgabe können auch mittels Enkapsulierung von dispergierten Mikropartikeln oder emulgierten Mikrotropfen über bekannte Beschichtungstechnologien von Dispersionen und Emulsionen erhalten werden.

Die Verbindungen der Formel I können auch zusammen mit wenigstens einem weiteren pharmazeutischen Wirkstoff für Kombinationstherapien verwendet werden. Hierzu kann wenigstens ein weiterer Wirkstoff zusätzlich in der erfindungsgemässen Zusammensetzung dispergiert oder gelöst werden.

Gegenstand der Erfindung ist auch die Verwendung von 3-[2-(Dimethylamino)methyl-(cyclohex-1-yl)]-phenol-Fumarat der Formel I zur Herstellung einer pharmazeutischen Zusammensetzung, insbesondere für die Behandlung von Schmerzzuständen.

Ein weiterer Gegenstand der Erfindung ist ein Verfahren zur Behandlung von Schmerzzuständen, bei dem man einem unter Schmerzen leidenden Patienten eine wirksame Menge von 3-[2-(Dimethylamino)methyl-(cyclohex-1-yl)]-phenol-Fumarat der Formel I verabreicht.

Bevorzugt eignet sich das erfindungsgemäße Arzneimittel (die erfindungsgemäße pharmazeutische Zusammensetzung) zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS): Panikattacken; Epilepsie; Husten; Harninkontinenz; Diarrhöe; Pruritus; Schizophrenie; cerebralen Ischämien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin-und/oder Kokain-) und/oder Medikamentenabhängigkeit vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; Entwicklung von Toleranzerscheinungen gegenüber Medikamenten; insbesondere gegenüber Opioiden; des Magen-Ösaphagus-Reflux-Syndroms; zur Diurese; zur Antinatriurese; zur Beeinflussung des kardiovaskulären Systems; zur Anxiolyse; zur Vigilanzsteigerung; zur Libidosteigerung, zur Modulation der Bewegungsaktivität und zur Lokalanästhesie.

Besonders bevorzugt eignet sich das erfindungsgemäße Arzneimittel (die erfindungsgemäße pharmazeutische Zusammensetzung) zur Prophylaxe und/oder Behandlung von Schmerzen, vorzugsweise von akuten Schmerzen, chronischen Schmerzen, neuropathischen Schmerzen oder visceralen Schmerzen; Depressionen; Epilepsie; Morbus Parkinson; Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit; vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Medikamenten, insbesondere gegenüber Opioiden, oder zur Anxiolyse.

Ganz besonders bevorzugt eignet sich das erfindungsgemäße Arzneimittel (die erfindungsgemäße pharmazeutische Zusammensetzung) zur Prophylaxe und/oder Behandlung von Schmerzen, vorzugsweise von akuten Schmerzen, chronischen Schmerzen, neuropathischen Schmerzen oder visceralen Schmerzen.

Besonders bevorzugt ist die Verwendung wenigstens eines erfindungsgemäßen Salzes, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, sowie ggf. eines oder mehrerer pharmazeutisch verträglicher Hilfsstoffe zur Herstellung eines Arzneimittels zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz, von Migräne, Depressionen, neurodegenerativen Erkrankungen, vorzugsweise ausgewählt aus der Gruppe bestehend aus Morbus Parkinson, Morbus Alzheimer, Morbus Huntington und Multipler Sklerose, kognitiven Erkrankungen, vorzugsweise kognitiven Mangelzuständen, besonders bevorzugt des Aufmerksamkeit-Defizit-Syndroms (ADS), Panikattacken, Epilepsie, Husten, Harninkontinenz, Diarrhöe, Pruritus, Schizophrenie, cerebralen Ischämien, Muskelspasmen, Krämpfen, Störungen der Nahrungsaufnahme, vorzugsweise ausgewählt aus der Gruppe bestehend aus Bulimie, Kachexie, Anorexie und Fettleibigkeit, Alkohol- und/oder Drogen (insbesondere Nikotin- und/oder Kokain-) und/oder Medikamentenmißbrauch; Alkohol- und/oder Drogen (insbesondere Nikotin- und/oder Kokain-) und/oder Medikamentenabhängigkeit vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; Entwicklung von Toleranzerscheinungen gegenüber Drogen- und/oder Medikamenten, insbesondere gegenüber Opioiden, des Magen-Ösaphagus-Reflux-Syndroms, zur Diurese, zur Antinatriurese, zur Beeinflussung des kardiovaskulären Systems, zur Anxiolyse, zur Vigilanzsteigerung, zur Libidosteigerung, zur Modulation der Bewegungsaktivität und zur Lokalanästhesie.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen Salz, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, , enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Geeignete perkutane Applikationszubereitungen sind auch Depotzubereitungen in gelöster Form oder in einem Pflaster, gegebenenfalls unter Zusatz von die Hautpenetration fördernden Mitteln.

Oral oder perkutan anwendbare Zubereitungsformen können die jeweiligen erfindungsgemäßen Salze verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in"Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen Salze kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg Körpergewicht des Patienten wenigstens einer solchen Verbindung appliziert.

Die nachfolgenden Beispiele erläutern die Erfindung näher.

Bei allen DSC Messungen (wenn nicht anders angegeben) betragen die Aufheizraten 10°C / Minute; die angebenen Temperaturen sind Peakmaxima.

### A) Herstellung von (+)-(1R,2R)-3-[2-(Dimethylamino)methyl-(cyclohex-1-yl)]-phenol-Fumarat

### Beispiel A1: Herstellung als kristalline Form B

0,0694 g (+)-(1R,2R)-3-[2-(Dimethylamino)methyl-(cyclohex-1-yl)]-phenol werden in 0,50 ml Ethylacetat gelöst. Daneben wird eine zweite Lösung von 0,0356 g Fumarsäure in 20 ml Ethylacetat zubereitet. Die zweite Lösung wird bei 40 °C zur ersten Lösung getropft und unter Rühren vermischt, wobei sich sofort ein weisser Niederschlag bildet. Die Mischung wird unter Rühren auf 23 °C abgekühlt, wobei sich ein klebriger Rückstand bildet. Der Rückstand wird mehrmals auf 40 °C erwärmt und danach auf 5 °C abgekühlt, bis ein weisser Feststoff zurückbleibt. Dann filtriert man den Niederschlag über eine Glasfritte ab und trocknet den Rückstand, indem man während 2 Minuten Luft durchsaugt. Man erhält 0,0810 mg (78% der Theorie) (+)-(1R,2R)-3-[2-(Dimethylamino)methyl-(cyclohex-1-yl)]-phenol-Fumarat als weissen, kristallinen Feststoff mit einem Schmelzpunkt von etwa 176 °C und einer Schmelzenthalpie beträgt etwa 113 J/g, bestimmt mittels Differential-Scanning Kalorimetrie (DSC) bei einer Aufheizrate von 10 °C/Minute. Der kristalline Feststoff ist die polymorphe Form B, deren Röntgen-Beugungsbild in Figur 1 dargestellt ist. Das Raman-Spektrum ist in Figur 2 dargestellt.

Wenn man die doppelte Menge an (+)-(1 R,2R)-3-[2-(Dimethylamino)methyl-(cyclohex-1-yl)]-phenol einsetzt, wird ebenfalls nur das 1:1-Fumarat gebildet.

### Beispiel A2: Herstellung als kristalline Form B

0,81 g (7,0 mmol) Fumarsäure werden in 400 ml Essigsäureethylester gelöst und in eine Lösung von 1,64 g (7 mmol) (+)-(1 R,2R)-3-[2-(Dimethylamino)methyl-(cyclohex-1-yl)]-phenol in 10 ml Essigsäureethylester getropft. An der Eintropfstelle tritt eine Trübung auf. Es bildet sich aber kein fester Niederschlag. Es setzt sich zunächst ein Öl ab, das nur langsam kristallisiert. Das Öl wird von der Wand das Glaskolbens abgeschabt und die ölige Suspension bei 50 bis 60 °C am Rotationsverdampfer gehalten, wobei die Kristallisation voran schreitet. Beim Abkühlen im Kühlschrank setzt sich ein weisser, schmieriger Niederschlag ab. Diese Mischung wird erneut bei 50 °C am Rotationsverdampfer gehalten und dann 3 Tage im Kühlschrank aufbewahrt. Dann erwärmt man die Suspension nochmals auf 50 °C am Rotationsverdampfer und kühlt dann auf Raumtemperatur ab. Der nun kristalline Niederschlag wird abfiltriert, mit Essigsäureethylester gewaschen und dann im Luftstrom getrocknet. Die Ausbeute an stäbchenförmigen Kristallen beträgt 95,3% der Theorie. Die DSC Analyse ergibt ein endothermes Signal (Peak) bei 175,6 °C. Nach Trocknung im Vakuum (100 mbar) bei 50 °C ergeben sich folgende Eigenschaften: DSC Peaks bei 156,6 und 174,5 °C, Röntgen-Beugungsbild entspricht Form B.

### Beispiel A3: Herstellung als kristalline Form B

Man suspendiert 8,92 g Fumarsäure in 120 ml Essigsäureethylester und erwärmt die Suspension auf 55 bis 60 °C. 17,96 g (+)-(1R-,2R)-3-[2-(Dimethylamino)methyl-(cyclohex-1-yl)]-phenol werden bei 60 °C in 108 ml Essigsäureethylester gelöst und langsam zur Suspension getropft. An der Eintropfstelle bildet sich sofort ein Feststoff. Die Suspension wird nach vollständiger Zugabe der Lösung noch 4 Stunden bei 60 °C und einer Umdrehungszahl des Rührers von 700 Upm weiter gerührt. Dann erwärmt man noch eine Stunde auf 70 °C und lässt danach die Suspension unter Rühren auf Raumtemperatur abkühlen. Der kristalline Feststoff wird abfiltriert, mit Essigsäureethylester gewaschen, nochmals in Essigsäureethylester aufgeschlämmt, dann abfiltriert und an der Luft getrocknet. Die Ausbeute beträgt 26,69 g (99,3% der Theorie. Das Röntgen-Beugungsbild entspricht der Form B. Die DSC Analyse gibt ein endothermes Signal (Peak) bei 175,1°C.

### Beispiel A4: Herstellung von 3-[2-(Dimethylamino)methyl-(cyclohex-1-yl)]-phenol-Fumarat

### a) Herstellung

23,16 g 3-[2-(Dimethylamino)methyl-(cyclohex-1-yl)]-phenol (Reinheit gemäss HPLC-Analyse 75,9%) werden in einem 1l Kolben in 588 ml Essigsäureethylester gelöst und die Lösung auf 55 °C erwärmt. Dann gibt man unter Rühren 8,75 g Fumarsäure auf einmal zu. Gebildete Agglomerate von Fumarsäure werden mit einem Spatel zerkleinert. Man rührt dann 4 Stunden bei 55 °C und nach dem Abkühlen noch 2 Stunden bei 20 °C. Danach wird der kristalline Niederschlag über eine Filterfritte abfiltriert, einmal mit 20 ml Essigsäureethylester gewaschen und mittels Durchsaugen von Luft getrocknet. Bei der Thermoanalyse (DSC) wird ein breites endothermes Signal bei 153,8 °C gefunden. Die Reinheit (bezogen of das Phenol) beträgt gemäss HPLC Analyse 89,4 %. Die Ausbeute beträgt 18,4 g (70,0% der Theorie).

### b) Reinigung mittels Rührwaschens

Das nach A4 a) hergestellte Rohprodukt wird in Glaskolben eingewogen, mit Lösungsmittel versetzt und bei 40 °C für 90 Minuten mit Ultraschall (100% Schallfrequenz) behandelt. Dann wird eine Stunde bei 4 °C gelagert und dann der gebildete Feststoff abgesaugt. Die an Luft getrockneten Rückstände werden zur Bestimmung der Ausbeute gewogen und die Reinheit mit HPLC Analyse bestimmt. Die Ergebnisse finden sich in nachfolgender Tabelle 1. Es werden folgende Abkürzungen verwendet: EtAc ist Ethylacetat, EtOH ist Ethanol, PrOH ist i-Propanol, AcNi ist Acetonitril, Tol ist Toluol, Hex ist n-Hexan, DiEt ist Diethylether, BuMe ist t-Butylmethylether, Ace ist Aceton, MeEt ist Methylethylketon, THF ist Tetrahydrofuran.

**Tabelle 1:**

| Menge Fumarat (mg) | Lösungsmittel 1 (ml) | Lösungsmittel 2 (ml) | Ausbeute (%) | Reinheit (%) |
|---|---|---|---|---|
| 100,6 | EtAc (2,0) | - | 95,9 | 90,0 |
| 100,6 | PrOH (2,0) | - | 66,2 | 94,9 |
| 100,7 | Hex (2,0) | - | 87,6 | 89,8 |
| 102,2 | AcNi (2,0) | - | 96,0 | 90,5 |
| 100,9 | Tol (2,0) | - | 99,5 | 89,5 |
| 100,3 | DiEt (2,0) | - | 73,9 | 89,9 |
| 101,1 | BuMe (2,0) | - | 92,4 | 89,9 |
| 101,4 | Ace (2,0) | - | 84,8 | 91,6 |
| 101,3 | MeEt (2,0) | - | 90,1 | 91,2 |
| 100,7 | THF (2,0) | - | 73,7 | 94,2 |
| 101,4 | Ace (1,98) | EtOH (0,02) | 76,2 | 92,8 |
| 101,0 | Ace (1,96) | EtOH (0,04) | 77,3 | 92,8 |
| 102,7 | Ace (1,94) | EtOH (0,06) | 72,2 | 94,1 |
| 101,7 | Ace (1,92) | EtOH (0,08) | 75,1 | 93,0 |
| 102,1 | Ace(1,90) | EtOH (0,10) | 74,2 | 93,4 |

### c) Reinigung mittels Rührwaschens

Das nach A4 a) erhaltene Rohprodukt wird in Glaskolben eingewogen, mit Lösungsmittel versetzt und in einem Thermomixer bei 50°C für 6 Stunden geschüttelt. Dann werden die Lösungen über Nacht bei 23 °C gelagert, filtriert, der Rückstand mit 2,5 ml des jeweiligen Lösungsmittels gewaschen und dann an der Luft getrocknet. Die Ausbeute und die Reinheit (HPLC) des Rückstandes werden bestimmt. Weitere Angaben finden sich in nachfolgender Tabelle 2.

**Tabelle 2:**

| Menge Fumarat (mg) | Lösungsmittel 1 (ml) | Lösungsmittel 2 (ml) | Ausbeute (%) | Reinheit (%) |
|---|---|---|---|---|
| 517,1 | PrOH (10,0) | - | 59,8 | 97,8 |
| 516,4 | Ace (10,0) | - | 73,2 | 92,7 |
| 506,9 | THF (10,0) | - | 67,6 | 96,6 |
| 518,3 | Ace (9,70) | EtOH (0,30) | 69,1 | 96,4 |

### d) Reinigung mittels Rührwaschens

Das nach A4 a) erhaltene Rohprodukt wird in Glaskolben eingewogen, mit Lösungsmittel versetzt und in einem Thermomixer bei 50 °C für 6 Stunden geschüttelt. Dann werden die Lösungen über Nacht bei 4 °C gelagert, filtriert, der Rückstand mit 2,5 ml des jeweiligen Lösungsmittels gewaschen und dann an der Luft getrocknet. Die Ausbeute und die Reinheit (HPLC) des Rückstandes werden bestimmt. Weitere Angaben finden sich in nachfolgender Tabelle 3.

**Tabelle 3:**

| Menge Fumarat (mg) | Lösungsmittel 1 (ml) | Lösungsmittel 2 (ml) | Ausbeute (%) | Reinheit (%) |
|---|---|---|---|---|
| 516,5 | PrOH (10,0) | - | 62,8 | 97,4 |
| 511,2 | Ace (10,0) | - | 79,3 | 91,9 |
| 506,9 | THF (10,0) | - | 67,9 | 96,5 |
| 514,3 | Ace (9,70) | EtOH (0,30) | 68,4 | 96,3 |

### e) Reinigung mittels Rührwaschens

Das so erhaltene Rohprodukt wird in Glaskolben eingewogen, mit Lösungsmittel versetzt und in einem Thermomixer bei 50 °C für 6 Stunden geschüttelt. Dann werden die Proben in Intervallen von etwa 17 Minuten um etwa 5 °C bis auf 20 °C abgekühlt und die Proben anschliessend 3 Tage bei 4 °C gelagert, filtriert, der Rückstand mit 2,5 ml des jeweiligen Lösungsmittels gewaschen und dann an der Luft getrocknet. Die Ausbeute und die Reinheit (HPLC) des Rückstandes werden bestimmt. Weitere Angaben finden sich in nachfolgender Tabelle 4.

**Tabelle 4:**

| Menge Fumarat (mg) | Lösungsmittel 1 (ml) | Lösungsmittel 2 (ml) | Ausbeute (%) | Reinheit (%) |
|---|---|---|---|---|
| 517,1 | PrOH (10,0) | - | 62,8 | 97,8 |
| 516,4 | Ace (10,0) | - | 79,3 | 91,6 |
| 506,9 | THF (10,0) | - | 67,5 | 96,5 |
| 518,3 | Ace (9,70) | EtOH (0,30) | 68,7 | 96,4 |

### f) Reinigung durch Umkristallisation

500 mg des nach A4 a) erhaltenen Rohproduktes werden in einen Glaskolben gegeben und dann mit 16 ml eines Lösungsmittelgemischs aus 80 Vol.-% Ace und 20 Vol.-% PrOH versetzt. Man erhitzt am Rotationsverdampfer zum Rückfluss, versetzt mit weiteren 30 ml des Lösungsmittelgemisches und hält am Rotationsverdampfer, bis sich eine klare Lösung gebildet hat. Die Lösung wird über Nacht langsam auf 23 °C abgekühlt, der kristalline Niederschlag abfiltriert und an der Luft getrocknet. Man erhält 308,8 mg (61,8%) kristallines Produkt in einer Reinheit von 97.3%.

260 mg des kristallinen Produktes werden erneut in 20 ml eines Lösungsmittelgemischs aus 80 Vol.-% Ace und 20 Vol.-% PrOH suspendiert und dann für 3 Stunden am Rückfluss erhitzt. Anschliessend kühlt man langsam auf 23 °C ab. Der kristalline Niederschlag wird abfiltriert und an der Luft getrocknet. Man erhält 210,8 mg (42,2%) kristallines Produkt in einer Reinheit von 99,05%.

### g) Reinigung durch Umkristallisation

500 mg des nach A4 a) erhaltenen Rohproduktes werden in einen Glaskolben gegeben und dann mit 30 ml eines Lösungsmittelgemischs aus 60 Vol.-% Ace und 40 Vol.-% PrOH versetzt. Man erhitzt am Rückfluss bis zur Bildung einer klaren Lösung, schaltet die Heizung ab und lässt die Lösung dann über Nacht auf Raumtemperatur abkühlen. Der kristalline Niederschlag wird abfiltriert und an der Luft getrocknet. Man erhält 197,9 mg (39,6%) kristallines Produkt in einer Reinheit von 97,7%.

### Beispiel A5: Herstellung von 3-[2-(Dimethylamino)methyl-(cyclohex-1-yl)]-phenol-Fumarat

In einem Glaskolben werden 3-[2-(Dimethylamino)methyl-(cyclohex-1-yl)]-phenol (im Kopf von Tabelle 5 als Base bezeichnet) und Fumarsäure vorgelegt und dann das Lösungsmittel oder Lösungsmittelgemisch zugegeben. Dann schüttelt man für 6 Stunden in einem Heizschüttler bei 55 °C. Dann kühlt man innerhalb einer Stunde auf 30 °C ab und lässt über Nacht bei Raumtemperatur stehen. Der kristalline Niederschlag wird abfiltriert und dann an der Luft getrocknet. Die an Luft getrockneten Rückstände werden zur Bestimmung der Ausbeute gewogen und die Reinheit mit HPLC Analyse bestimmt. Die Ergebnisse finden sich in nachfolgender Tabelle 5. EtAc ist Ethylacetat. Die besten Reinheiten werden bei Zugabe von einem halben Äquivalent Fumarsäure (89,7 mg) beobachtet, allerdings bei nur niedrigen Ausbeuten.

**Tabelle 5:**

| Fumarsäure (mg) | Base (mg) | EtAc (ml) | PrOH (ml) | Ausbeute (%) | Reinheit (%) |
|---|---|---|---|---|---|
| 134,7 | 360,4 | 5,57 | 0,62 | 56,83 | 85,84 |
| 179,9 | 360,2 | 5,40 | 1,35 | 79,91 | 89,08 |
| 179,7 | 360,3 | 6,75, | - | 57,54 | 81,35 |
| 89,8 | 360,2 | 5,63 | - | 42,04 | 79,76 |
| 89,7 | 360,4 | 5,63 | - | 48,83 | 81,05 |
| 135,4 | 360,1 | 5,57 | 0,62 | 44,43 | 92,33 |
| 89,6 | 360,0 | 4,50 | 1,12 | 38,47 | 92,35 |
| 89,9 | 360,1 | 4,50 | 1,13 | 39,36 | 92,92 |
| 179,4 | 360,3 | 6,75 | - | 64,10 | 81,37 |
| 179,4 | 360,5 | 5,40 | 1,35 | 90,49 | 83,80 |
| 134,7 | 360,0 | 5,57 | 0,62 | 47,10 | 80,76 |

### B) Herstellung von amorphem (+)-(1R,2R)-3-[2-(Dimethylamino)methyl-(cyclohex-1-yl)]-phenol-Fumarat

### Beispiel B1: Gefriertrocknung

601,2 mg des gemäss Beispiel A1 hergestellten Fumarats werden in 8 ml Wasser gelöst und danach auf -85°C abgeschreckt. Dann wird bei dieser Temperatur und einem Druck von 0,250 mbar während 21 Stunden gefriergetrocknet. Man erhält quantitativ einen festen, weissen Rückstand, der in Gegenwart von 22% relativer Luftfeuchtigkeit und bei 25°C auch nach 2 Monaten noch amorph ist. Mittels Differential Scanning Calorimetrie (DSC, Erwärmungsrate 10°C/Minute) wird eine Glasumwandlungstemperatur nahe 55 °C ermittelt. Ein exothermer Peak bei 118°C wird auf eine Kristallisation zurückgeführt. Das gebildete kristalline Material schmilzt bei etwa 175,5 °C, was auf die Bildung der polymorphen Form A hinweist.

### Beispiel B2: Gefriertrocknung

500,7 mg des gemäss Beispiel A1 hergestellten Fumarats werden in 8 ml Wasser gelöst und danach auf -80 °C abgeschreckt. Dann wird bei dieser Temperatur und einem Druck von 0,570 mbar während 20 Stunden gefriergetrocknet. Man erhält quantitativ einen festen, weissen Rückstand, der amorph ist.

### C) Herstellung der kristallinen Form A

Die Kristallformen werden, wenn nicht anders angegeben, durch Vergleich der Raman-Spektren bestimmt.

### Beispiel C1: Phasengleichgewichte mit Suspensionen in Ethylacetat

Zu 101 mg des gemäss Beispiel A1 hergestellten Fumarats werden 25 ml Ethylacetat zugegeben und unter Rühren auf 70 °C erwärmt. Dann rührt man 2 Stunden bei dieser Temperatur. Danach wird auf Raumtemperatur abgekühlt, abfiltriert und in einem Luftstrom bei Raumtemperatur getrocknet. Man erhält 87 mg der kristallinen Form A als weisses Pulver.

### Beispiel C2: Kristallisation aus Ethylacetat

80 mg des gemäss Beispiel A1 hergestellten Fumarats werden bei 65 °C in 60 ml Ethylacetat gelöst. Die erhaltene klare Lösung wird zunächst auf Raumtemperatur abgekühlt und 2 Stunden bei dieser Temperatur gehalten. Es bildet sich ein weisser Niederschlag. Die Suspension lässt man noch 18 Stunden bei 5 °C stehen. Dann wird der Niederschlag abfiltriert und der Rückstand in einem Luftstrom bei Raumtemperatur getrocknet. Man erhält 55 mg der kristallinen Form A in Form eines weissen Pulvers. Der kristalline Feststoff weist einen Schmelzpunkt von 175,5 °C und eine Schmelzenthalpie von 111 J/g auf, bestimmt mittels Differential-Scanning Kalorimetrie (DSC) bei einer Aufheizrate von 10 °C/Minute. Der kristalline Feststoff ist die polymorphe Form A, deren Röntgen-Beugungsbild in Figur 3 dargestellt ist. Das Raman-Spektrum ist in Figur 4 dargestellt.

### Beispiel C3: Kristallisation aus Ethylacetat

400 mg des gemäss Beispiel A1 hergestellten Fumarats werden während 2 Stunden bei 65 °C in 450 ml Ethylacetat gelöst. Die erhaltene klare Lösung wird zunächst auf Raumtemperatur abgekühlt und 2,5 Stunden bei dieser Temperatur gehalten, wobei sich kein Niederschlag bildet. Die Lösung lässt man noch 4 Tage bei 5 °C stehen.

Dann wird der gebildete weisse Niederschlag abfiltriert und der Rückstand in einem Luftstrom bei Raumtemperatur getrocknet. Man erhält 301 mg der kristallinen Form A in Form eines weissen Pulvers.

### Beispiel C4: Kristallisation aus Tetrahydrofuran (THF)

50 mg des gemäss Beispiel A1 hergestellten Fumarats werden während 45 Minuten bei 60 °C in 4 ml THF gelöst. Dann kühlt man mit Trockeneis, wobei sich kein Niederschlag bildet. Man lässt noch 18 Tage bei -18 °C stehen. Dann wird der gebildete weisse Niederschlag abfiltriert und der Rückstand in einem Luftstrom bei Raumtemperatur getrocknet. Man erhält 12 mg der kristallinen Form A in Form eines weissen Pulvers.

Beispiel C5: Kristallisation aus Isopropanol mittels Verdampfung 29 mg des gemäss Beispiel B2 hergestellten amorphen Fumarats werden in 6 ml Isopropanol bei Raumtemperatur gelöst und durch einen 0,22 µm Filter filtriert. Die Lösung wird danach 2 Tage bei 50 °C im offenen Gefäss gerührt. Dann wird auf Raumtemperatur abgekühlt und das restliche Lösungsmittel im Stickstoffstrom verdampft. Bei dem festen Rückstand handelt es sich um die kristalline Form A.

### D) Herstellung der kristallinen Form B

### Beispiel D1:

80,1 mg des gemäss Beispiel B1 hergestellten amorphen Fumarats werden in 2 ml Aceton suspendiert und bei 23 °C für 24 Stunden gerührt. Dann wird abfiltriert und der Rückstand in einem Luftstrom bei Raumtemperatur getrocknet. Man erhält 50 mg der kristallinen Form B in Form eines weissen Pulvers. Schmelzpunkt, Schmelzenthalpie, Röntenbeugungsbild und Raman-Spektrum entsprechen den in Beispiel A1 angegeben Werten, Bild und Spektrum.

### Beispiel D2:

80 mg des gemäss Beispiel B1 hergestellten amorphen Fumarats werden in 2 ml Dioxan suspendiert und bei 23 °C für 24 Stunden gerührt. Dann wird abfiltriert und der Rückstand in einem Luftstrom bei Raumtemperatur getrocknet. Man erhält 35 mg der kristallinen Form B in Form eines weissen Pulvers.

### Beispiel D3:

80 mg des gemäss Beispiel B1 hergestellten amorphen Fumarats werden in 2 ml Ethylacetat suspendiert und bei 23 °C für 24 Stunden gerührt. Dann wird abfiltriert und der Rückstand in einem Luftstrom bei Raumtemperatur getrocknet. Man erhält 50 mg der kristallinen Form B in Form eines weissen Pulvers.

### Beispiel D4:

80 mg des gemäss Beispiel B1 hergestellten amorphen Fumarats werden in 2,5 ml Isopropanol suspendiert und bei 23 °C für 24 Stunden gerührt. Dann wird abfiltriert und der Rückstand in einem Luftstrom bei Raumtemperatur getrocknet. Man erhält 36 mg der kristallinen Form B in Form eines weissen Pulvers.

### Beispiel D5:

80 mg des gemäss Beispiel B1 hergestellten amorphen Fumarats werden in 2 ml Tetrahydrofuran suspendiert und bei 23 °C für 24 Stunden gerührt. Dann wird abfiltriert und der Rückstand in einem Luftstrom bei Raumtemperatur getrocknet. Man erhält 44 mg der kristallinen Form B in Form eines weissen Pulvers.

### Beispiel D6:

80 mg des gemäss Beispiel B1 hergestellten amorphen Fumarats werden in 2 ml Tertiärbutylmethylether suspendiert und bei 23 °C für 24 Stunden gerührt. Dann wird abfiltriert und der Rückstand in einem Luftstrom bei Raumtemperatur getrocknet. Man erhält 58 mg der kristallinen Form B in Form eines weissen Pulvers.

### Beispiel D7:

40 mg des gemäss Beispiel B1 hergestellten amorphen Fumarats werden in 0,02 ml Wasser suspendiert und bei 23 °C für 24 Stunden gerührt. Dann wird abfiltriert und der Rückstand in einem Luftstrom bei Raumtemperatur getrocknet. Man erhält 18 mg der kristallinen Form B in Form eines weissen Pulvers.

### Beispiel D8:

25 mg des gemäss Beispiel A1 hergestellten Fumarats und 25 mg des gemäss Beispiel C3 hergestellten Fumarats werden in 1 ml Isopropanol suspendiert und bei 23 °C für 24 Stunden gerührt. Dann wird abfiltriert und der Rückstand in einem Luftstrom bei Raumtemperatur getrocknet. Man erhält 31 mg der kristallinen Form B in Form eines weissen Pulvers.

### Beispiel D9:

24 mg des gemäss Beispiel D2 hergestellten Fumarats und 20 mg des gemäss Beispiel A1 hergestellten Fumarats werden in 5 ml Isopropanol suspendiert und bei 23 °C für 19 Stunden gerührt. Dann wird abfiltriert und der Rückstand in einem Luftstrom bei Raumtemperatur getrocknet. Man erhält 3,3 mg der kristallinen Form B in Form eines weissen Pulvers.

### Beispiel D10:

20 mg des gemäss Beispiel B2 hergestellten amorphen Fumarats werden in 2 ml Ethylacetat suspendiert und bei 23 °C für 1 Monat gerührt. Dann wird abfiltriert und der Rückstand in einem Luftstrom bei Raumtemperatur getrocknet. Man erhält 12 mg der kristallinen Form B in Form eines weissen Pulvers.

### Beispiel D11:

20 mg des gemäss Beispiel B2 hergestellten amorphen Fumarats werden in 2 ml Ethylacetat suspendiert und bei 40 °C für 1 Monat gerührt. Dann wird abfiltriert und der Rückstand in einem Luftstrom bei Raumtemperatur getrocknet. Man erhält 11 mg der kristallinen Form B in Form eines weissen Pulvers.

### Beispiel D12:

20 mg des gemäss Beispiel B2 hergestellten amorphen Fumarats werden in 2 ml Toluol suspendiert und bei 40 °C für 1 Monat gerührt. Dann wird abfiltriert und der Rückstand in einem Luftstrom bei Raumtemperatur getrocknet. Man erhält 6 mg der kristallinen Form B in Form eines weissen Pulvers.

### Beispiel D13: Umkristallisation aus Isopropanol

In einem Rührkolben werden 2,0 g des gemäss Beispiel A4a hergestellten Fumaratsalzes in 30 ml Isopropanol suspendiert und innerhalb 10 Minuten bei einer Umdrehungszahl des Rührers von etwa 600 Upm (Umdrehungen pro Minute) auf 80 °C unter Bildung einer klaren Lösung erhitzt. Man erhöht die Umdrehungszahl auf 800 Upm und rührt bei dieser Temperatur für 10 Minuten, wobei gegen Ende die Umdrehungszahl kurz auf 1000 Upm erhöht wird. Dann wird innerhalb von 155 Minuten die Temperatur mit zunehmender Kühlrate auf 10 °C gesenkt. Der weisse Feststoff wird abfiltriert und an der Luft getrocknet (1,315 g, 65,8%). Gemäss Röntgen-Pulverdiffraktogramm werden nur Linien der kristallinen Form B gemessen. Die Thermoanalyse (DSC) zeigt endotherme Signale bei 152,4 °C und 174,0 °C. Bei der thermogravimetrischen Analyse wird kein signifikanter Gewichtsverlust beobachtet, so dass keine solvatisierte Form vorliegen kann. Die Reinheit beträgt, bestimmt mit HPLC, 97,9%.

### Beispiel D13: Umkristallisation aus Isopropanol

In einem Rührkolben werden 2,0 g des gemäss Beispiel A4a hergestellten Fumaratsalzes in 30 ml Isopropanol suspendiert und innerhalb 10 Minuten bei einer Umdrehungszahl des Rührers von etwa 600 Upm (Umdrehungen pro Minute) auf 80 °C unter Bildung einer klaren Lösung erhitzt. Man erhöht die Umdrehungszahl auf 800 Upm und rührt bei dieser Temperatur für 10 Minuten, wobei gegen Ende die Umdrehungszahl kurz auf 1000 Upm erhöht wird. Dann wird innerhalb von 140 Minuten die Temperatur mit einer linearen Kühlrate von 0,2 K/min auf 10 °C gesenkt. Der weisse Feststoff wird abfiltriert und an der Luft getrocknet (1,318 g, 65,9%). Gemäss Röntgen-Pulverdiffraktogramm werden nur Linien der kristallinen Form B gemessen. Die Thermoanalyse (DSC) zeigt endotherme Signale bei 151,9 °C und 174,2 °C. Bei der thermogravimetrischen Analyse wird kein signifikanter Gewichtsverlust beobachtet, so dass keine solvatisierte Form vorliegen kann. Die Reinheit beträgt, bestimmt mit HPLC, 98,0%.

### E) Stabilitätsuntersuchungen

### Beispiel E1: Lagerung der amorphen Form in hoher und niedriger Luftfeuchtigkeit

a) 30 mg des gemäss Beispiel B1 hergestellten amorphen Fumarats werden bei Raumtemperatur und 75% relativer Luftfeuchtigkeit für 5 Tage gelagert. Danach ist das amorphe Produkt vollständig in die kristalline Form B umgewandelt.
b) 30 mg des gemäss Beispiel B2 hergestellten amorphen Fumarats werden bei Raumtemperatur und 90% relativer Luftfeuchtigkeit für 3 Tage gelagert. Danach ist das amorphe Produkt vollständig in die kristalline Form B umgewandelt.
c) 30 mg des gemäss Beispiel B2 hergestellten amorphen Fumarats werden bei Raumtemperatur und 53% relativer Luftfeuchtigkeit für 2 Monate gelagert. Danach ist das amorphe Produkt vollständig in die kristalline Form B umgewandelt.
d) 30 mg des gemäss Beispiel B2 hergestellten amorphen Fumarats werden bei Raumtemperatur und 22% relativer Luftfeuchtigkeit für 2 Monate gelagert. Danach liegt immer noch das amorphe Produkt vor.

### Beispiel E2: Lagerung der kristallinen Form A in hoher Luftfeuchtigkeit

Das gemäss Beispiel C3 hergestellte Fumarat wird bei Raumtemperatur und 75% relativer Luftfeuchtigkeit gelagert und nach 3 Tagen, 1, 2, 3, 4 und 8 Wochen werden Proben mittels Raman Spektroskopie untersucht. Die kristalline Form A ist schon nach 3 Tagen in die kristalline Form B umgewandelt und auch nach 8 Wochen liegt die kristalline Form B vor.

### Beispiel E3: Lagerung der kristallinen Form B in hoher Luftfeuchtigkeit

Die gemäss Beispiel A1 hergestellte kristalline Form B wird bei Raumtemperatur und 75% relativer Luftfeuchtigkeit gelagert und nach 3 Tagen, 1, 2, 3, 4 und 8 Wochen werden Proben mittels Raman Spektroskopie untersucht. Die Proben sind auch nach 8 Wochen praktisch unverändert.

### Beispiel E4: Stabilität unter Malbedingungen

a) Kristalline Form A beim Verreiben Das gemäss Beispiel C3 hergestellte Fumarat wird in einer Mörserschale mit einem Stössel 5 Minuten lang verrieben. Danach liegt eine Mischung der kristallinen Form A und B vor.
b) Kristalline Form B beim Verreiben
   Die kristalline Form B gemäss Beispiel A1 wird in einer Mörserschale mit einem Stössel 5 Minuten lang verrieben. Danach liegt unverändert die kristalline Form B vor.
c) Kristalline Form A beim Mahlen
   Das gemäss Beispiel A1 hergestellte Fumarat wird in eine Achat-Kugelmühle gefüllt (Retsch MM200 Mischmühle mit 5 mm Achatkugel) und bei 20 Hz und Raumtemperatur 180 Minuten gemahlen. Danach liegt eine Mischung der kristallinen Form A und B vor.
c) Kristalline Form B beim Mahlen
   Mit der kristallinen Form B gemäss Beispiel A1 wird gemäss Beispiel E4c) verfahren. Nach 180 Minuten liegt ein Gemisch der kristallinen Formen A und B vor.

### Beispiel E5: Stabilität unter Druck

a) Kristalline Form A beim Pressen von Tabletten Das gemäss Beispiel C3 hergestellte Fumarat wird in eine Tablettenpresse gefüllt und unter Vakuum bei einem Druck von 100 MPa für 60 Minuten zu einer Tablette gepresst. Danach liegt ein Gemisch der kristallinen Formen A und B vor.
b) Kristalline Form B beim Pressen von Tabletten Mit der kristallinen Form B gemäss Beispiel A1 wird gemäss Beispiel E5a) verfahren. Nach 60 Minuten liegt ein Gemisch der kristallinen Formen A und B vor.

### Beispiel E6: Wasseraufnahme

Die Wasseraufnahme wird mittels dynamischer Wasserdampfaufnahme (Dynamic Vapor Sorption, DVS) mit dem Gerät DVS-1 der Firma Surface Measurement Systems Ltd.emittelt. Die Probe wird in einem Platintiegel an der Spitze einer Mikrowaage plaziert. Dann wird die Probe zunächst bei 50% relativer Luftfeuchtigkeit äquilibriert und dann einem vordefinierten Messprogramm unterzogen. Die Temperatur beträgt 25 °C. Bestimmt wird die Gewichtsänderung der Probe.
a) Amorphe Form
   Die amorphe Form weist bis etwa 77% relative Luftfeuchtigkeit eine ausgeprägte Wasseraufnahme von etwa 6,5 Gew.-% auf. Bei höherer Luftfeuchtigkeit fällt das Gewicht wieder ab, was durch eine Kristallisation verursacht wird. Nach Ende des Versuchs liegt die kristalline Forme A vor.
b) Kristalline Form A
   Die kristalline Form A weist eine Wasseraufnahme von lediglich etwa 0,2 Gew.-%. Am Ende des Messzyklus liegt im wesentlichen die kristalline Form B vor.
c) Kristalline Form B
   Die kristalline Form B weist eine Wasseraufnahme von lediglich etwa 0,2 Gew.-% auf. Am Ende des Messzyklus liegt unverändert die kristalline Form B vor.

### Beispiel E7: Stabilität bei erhöhter Temperatur

Die kristallinen Formen werden in offenen und geschlossen Gefässen gelagert und nach einer vorgegebenen Zeit mögliche Veränderungen chromatographisch (HPLC) als Stabilitätsparameter gemessen. Die kristalline Form A erweist sich hinsichtlich chemischer Stabilität und Kristallform als sehr stabil. Die kristalline Form B erweist sich hinsichtlich chemischer Stabilität als sehr stabil. Die Ergebnisse sind in der nachfolgenden Tabelle 6 angegeben.

**Tabelle 6:**

| Polymorph | Bedingungen | Zeit | HPLC | FT Raman¹⁾ |
|---|---|---|---|---|
| B | 40 °C/75% r.L. | 1 Woche | 100,0% | B |
| B | 60 °C/75% r.L. | 4 Wochen | 99,1% | B |
| B | 60°C (geschlossen) | 1 Woche | 99,0% | B |
| B | 60 °C (geschlossen) | 4 Wochen | 100,6% | B |
| B | -18 °C (Referenz) | 4 Wochen | 100% | B |
| A | 40 °C/75% r.L. | 1 Woche | 99,5% | B |
| A | 60 °C/75% r.L. | 4 Wochen | 99,3% | B |
| A | 60 °C (geschlossen) | 1 Woche | 99,9% | A und B |
| A | 60 °C (geschlossen) | 4 Wochen | 99,6% | A und B |
| A | -18 °C (Referenz) | 4 Wochen | 100% | A und B |

| | | | | |
|---|---|---|---|---|
| ¹) Bestimmung kristalline Form | | | | |

### Beispiel E8: Wasseraufnahme von Fumarat und Hydrochlorid (Vergleich)

Die Wasseraufnahme wird mittels dynamischer Wasserdampfaufnahme (Dynamic Vapor Sorption, DVS) mit dem Gerät DVS-1 der Firma Surface Measurement Systems Ltd. Ermittelt. Die Probe wird in einem Platintiegel an der Spitze einer Mikrowaage plaziert. Dann wird die Probe zunächst bei 50% relativer Luftfeuchtigkeit äquilibriert und dann einem vordefinierten Messprogramm unterzogen. Die Temperatur beträgt 25 °C. Bestimmt wird die Gewichtsänderung der Probe nach einer schrittweisen Erhöhung der relativen Luftfeuchtigkeit um je 10% bis auf 90%.

### a) (+)-(1R,2R)-3-(2-(Dimethylamino)methyl-(cyclohex-1-yl)1-phenol-Fumarat (Form B)

Es wird eine ausgesprochen geringe Wasseraufnahme von 0,13% gefunden. Die absorbierte Feuchte wird bei etwa 10% relativer Luftfeuchtigkeit wieder vollständig abgegeben.

### b) (+)-(1R,2R)-3-[2-(Dimethylamino)methyl-(cydohex-1-yl)]-phenol-Hydrochlorid Form D)

Bei einer Erhöhung der relativen Luftfeuchtigkeit bis etwa 75% wird eine Wasseraufnahme von etwa 2 Gew.-% beobachtet. Eine weitere Erhöhung bis 90% relativer Luftfeuchtigkeit führt zu einer Wasseraufnahme von gesamt etwa 5 Gew.-%. Bei der Desorption durch schrittweise Erniedrigung der relativen Luftfeuchtigkeit um 10% wird mehr Wasser abgegeben als zuvor aufgenommen, nämlich insgesamt 8,5%. Es wird vermutet, dass ein variables Hydrat vorliegt.

### Beispiel E9: Feuchtlagerung und Trocknung von Fumarat und Hydrochlorid (Vergleich)

Fumarat und Hydrochlorid gemäss Beispiel E8 werden zunächst bei 25 °C und 75% und 95% relativer Luftfeuchtigkeit 7 Tage gelagert und danach 7 Tage im Trockenschrank bei 50 °C im Vakuum getrocknet. Vor Beginn der Lagerung werden Röntgenbeugungsbilder (XRD), DSC zur Bestimmung von Übergangspunkten (Tp), des Trockengewichts (TG) und der Wassergehalt nach Karl-Fischer (WG) bestimmt und die gleichen Werte nach der Feuchtlagerung und Trocknung. Die Röntgenbeugungsbilder zeigen keine Veränderung der kristallinen Formen A und D nach Feuchtlagerung und Trocknung. Die Ausgangswerte für das Hydrochlorid sind Tp = 116,27 °C, TG = 6,35% und WG 8,00%. Die Ausgangswerte für das Fumarat sind Tp = 93,11 °C und 175.14 °C, TG = 0 und WG = 0,2%. Die Ergebnisse sind in Tabellen 7 (75% relative Luftfeuchtigkeit) und 8 (95% relative Luftfeuchtigkeit) angegeben.

**Tabelle 7 (75%):**

| Salz | Tp (°C) | TG (%) | WG (%) | Tp (°C) | TG (%) | WG (%) |
|---|---|---|---|---|---|---|
| Fumarat | 91,97/175,14 | - | 0,50 | 92,67/174/83 | - | 0,30 |
| Hydrochlorid | 116,22 | 5,92 | 8,50 | 113,83 | 6,12 | 6,00 |

**Tabelle 8 (95%):**

| Salz | Tp (°C) | TG (%) | WG (%) | Tp (°C) | TG | WG (%) |
|---|---|---|---|---|---|---|
| Fumarat | 92,63/175,20 | - | 0,50 | 92,67/174,89 | -- | 0,30 |
| Hydrochlorid | 116,52 | 7,84 | 8,50 | 114,47 | 6,74 | 6,60 |

### Geräte, Methoden:

Differential Scanning Calorimetrie (DSC): Gerätebezeichnung Perkin Elmer DSC 7 oder Perkin Elmer Pyris 1. Variable Messungen (Heizungsrate) in Gold- oder Aluminiumtiegeln.

Pulver Röntgenstrahlung Beugungsbilder (PXRD):

PXRD wird mit einem Philips 1710 Pulver X-Strahlung Diffraktometer durchgeführt, wobei CuK_{α}-Strahlung verwendet wird. D-Abstände werden von den 2θ Werten berechnet, wobei die Wellenlänge von 1.54060 Å zu Grunde gelegt ist. Es gilt allgemein, dass die 2θ Werte eine Fehlerrate von ±0.1-0.2° besitzen. Der experimentelle Fehler bei den d-Abstandswerten ist daher abhängig vom Ort der Linie (des Peaks).

### Raman Spektroskopie:

FT-Raman Spektren werden mit einem Bruker RFS 100 FT-Raman System aufgenommen, der mit einem Nd:YAG Laser (Wellenlänge 1064 nm) und einem mit flüssigen Stickstoff gekühltem Germanium Detektor betrieben wird. Für jede Probe werden 64 Abtastungen mit einer Auflösung von 2 cm⁻¹ akkumuliert. Generell wird eine Laserleistung von 100 mW verwendet.

### Beschreibung der Figuren

Figur 1 zeigt das Röntgen-Beugungsbild der polymorphen Form B
Figur 2 zeigt das Raman-Spektrum der polymorphen Form B
Figur 3 zeigt das Röntgen-Beugungsbild der polymorphen Form A
Figur 4 zeigt das Raman-Spektrum der polymorphen Form A

## Patentansprüche

1. Salze von Fumarsäure und 3-[2-(Dimethylamino)methyl-(cyclohex-1-yl)]-phenol der
Formel I

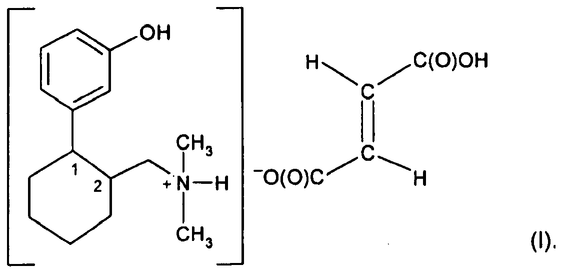

2. Salze gemäß Anspruch 1, **dadurch gekennzeichnet, dass** die Verbindung der Formel I als Diastereomer oder Gemisch von enantiomeren Diastereomeren mit Transkonfiguration des Phenylringes und der Dimethylaminomethylgruppe (1 R,2R-beziehungsweise 1S,2S-Konfiguration) vorliegt.

3. Salze gemäß Anspruch 2, **dadurch gekennzeichnet, dass** die Verbindung der Formel I als Enantiomer mit der absoluten Konfiguration (1 R,2R) vorliegt.

4. Salze gemäß Anspruch 1, **dadurch gekennzeichnet, dass** 3-[2-(Dimethylamino)methyl-(cyclohex-1-yl)]-phenol-Fumarat der Formel I gemäß Anspruch 1 in einer kristallinen Form B vorliegt, die
ein charakteristisches Röntgen-Beugungsbild im Bereich von 2° bis 35° 2θ mit ausgeprägten charakteristischen Linien aufweist, ausgedrückt in d-Werten (Å): 9,3 (vs), 7,0 (m), 6.7 (s), 5,37 (s), 5,21 (s), 4,64 (s), 4,52 (s), 4,28 (vs), 4,23 (s), 4,19 (s), 3,94 (m) 3,78 (m), 3,52 (m), 3,49 (m), 3,33 (s), 3,30 (m), 3,06 (s), 2,83 (m) aufweist.

5. Salze gemäß Anspruch 4, **dadurch gekennzeichnet, dass** 3-[2-(Dimethylamino)methyl-(cyclohex-1-yl)]-phenol-Fumarat in der Konfiguration (1R, 2R) vorliegt.

6. Salze gemäß Anspruch 1, **dadurch gekennzeichnet, dass** 3-[2-(Dimethylamino)methyl-(cyclohex-1-yl)]-phenol-Fumarat der Formel I gemäß Anspruch 1 in einer kristallinen Form A vorliegt, die
ein charakteristisches Röntgen-Beugungsbild im Bereich von 2° bis 35° 2θ mit ausgeprägten charakteristischen Linien aufweist, ausgedrückt in d-Werten (Å): 9,3 (vs), 7,0 (m), 6,7 (s), 5,36 (vs), 5,20 (m) 4,64 (vs), 4,53 (s), 4,26 (vs), 4,18 (s), 3,95 (m), 3,78 (m), 3,57 (m), 3,50 (s), 3,46 (m), 3,33 (s), 3,05 (m), 2,83 (m),
aufweist.

7. Salze gemäß Anspruch 6, **dadurch gekennzeichnet, dass** 3-[2-(Dimethylamino)methyl-(cyclohex-1-yl)]-phenol-Fumarat in der Konfiguration (1 R, 2R) vorliegt.

8. Verfahren zur Herstellung von 3-[2-(Dimethylamino)methyl-(cyclohex-1-yl)]-phenol-fumarat der Formel I gemäß Anspruch 1, umfassend die Vereinigung von 3-[2-(Dimethylamino)methyl-(cyclohex-1-yl)]-phenol und Fumarsäure.

9. Verfahren gemäß Anspruch 8 zur Herstellung von 3-[2-(Dimethylamino)methyl-(cyclohex-1-yl)]-phenol-Fumarat der Formel I gemäß Anspruch 1,
umfassend die Schritte
a) Lösen oder Suspendieren von 3-[2-(Dimethylamino)methyl-(cyclohex-1-yl)]-phenol in einem Lösungsmittel, oder Vorlegen von 3-[2-(Dimethylamino)methyl-(cyclohex-1-yl)]-phenol in Substanz,
b) Vermischen der Lösung, Suspension oder Substanz mit Fumarsäure, einer Suspension oder einer Lösung von Fumarsäure in einem Lösungsmittel, gegebenenfalls Abkühlen und Halten bei niedriger Temperatur,
c) Isolieren der Verbindung der Formel I,
wobei die Schritte a) und b) auch vertauscht sein können.

10. Pharmazeutische Zusammensetzung, enthaltend eine wirksame Menge 3-[2-(Dimethylamino)methyl-(cyclohex-1-yl)]-phenol-Fumarat der Formel I gemäß Anspruch 1 und einen pharmazeutischen Träger oder ein pharmazeutisches Verdünnungsmittel.

11. Zusammensetzung gemäss Anspruch 10, worin die Verbindung der Formel I als kristalline Form A, kristalline Form B oder in einer Mischung der Formen A und B vorliegt.

12. Zusammensetzung gemäss Anspruch 11, worin die Verbindung der Formel I als kristalline Form B enthalten ist.

13. Verwendung von 3-[2-(Dimethylamino)methyl-(cyclohex-1-yl)]-phenol-Fumarat der Formel I zur Herstellung einer pharmazeutischen Zusammensetzung.

14. Verwendung gemäß Anspruch 13 zur Herstellung einer pharmazeutischen Zusammensetzung für die Behandlung von Schmerzzuständen.

## Claims

1. Salts of fumaric acid and 3-[2-(dimethylamino)methyl-(cyclohex-1-yl)]-phenol of formula I

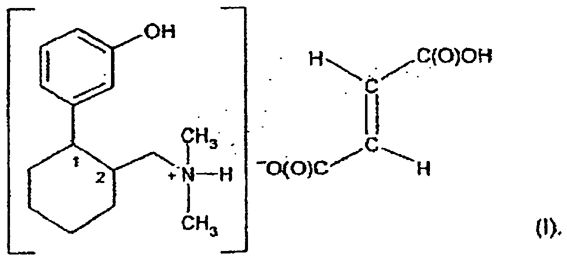

2. Salts according to claim 1, **characterised in that** the compound of formula I is in the form of a diastereoisomer or mixture of enantiomeric diastereoisomers with transconfiguration of the phenyl ring and of the dimethylaminomethyl group (1R,2R or 1S,2S configuration).

3. Salts according to claim 2, **characterised in that** the compound of formula I is in the form of the enantiomer having the absolute configuration (1R,2R).

4. Salts according to claim 1, **characterised in that** 3-[2-(dimethylamino)methyl-(cyclohex-1-yl)]-phenol fumarate of formula I is in a crystalline form B which
exhibits a characteristic X-ray diffraction pattern in the range from 2° to 35° 2θ with pronounced characteristic lines, expressed in d-values (Å) : 9.3 (vs), 7.0 (m), 6.7 (s), 5.37 (s), 5.21 (s), 4.64 (s), 4.52 (s), 4.28 (vs), 4.23 (s), 4.19 (s), 3.94 (m), 3.78 (m), 3.52 (m), 3.49 (m), 3.33 (s), 3.30 (m), 3.06 (s), 2.83 (m).

5. Salts according to claim 4, **characterised in that** 3-[2-(dimethylamino)methyl-(cyclohex-1-yl)]-phenol fumarate is present in the (1R,2R) configuration.

6. Salts according to claim 1, **characterised in that** 3-[2-(dimethylamino)methyl-(cyclohex-1-yl)]-phenol fumarate of formula I according to claim 1 is present in a crystalline form A which
exhibits a characteristic X-ray diffraction pattern in the range from 2° to 35° 2θ with pronounced characteristic lines, expressed in d-values (Å): 9.3 (vs), 7.0 (m), 6.7 (s), 5.36 (vs), 5.20 (m), 4.64 (vs), 4.53 (s), 4.26 (vs), 4.18 (s), 3.95 (m), 3.78 (m), 3.57 (m), 3.50 (s), 3.46 (m), 3.33 (s), 3.05 (m), 2.83 (m).

7. Salts according to claim 6, **characterised in that** 3-[2-(dimethylamino)methyl-(cyclohex-1-yl)]-phenol fumarate is present in the (1R,2R) configuration.

8. Process for the preparation of 3-[2-(dimethylamino)-methyl-(cyclohex-1-yl)]-phenol fumarate of formula I according to claim 1, which comprises combining 3-[2-(dimethylamino)methyl-(cyclohex-1-yl)]-phenol and fumaric acid.

9. Process according to claim 8 for the preparation of 3-[2-(dimethylamino)methyl-(cyclohex-1-yl)]-phenol fumarate of formula I according to claim 1, comprising the steps
a) dissolving or suspending 3-[2-(dimethylamino)-methyl-(cyclohex-1-yl)]-phenol in a solvent, or placing 3-[2-(dimethylamino)methyl-(cyclohex-1-yl)]-phenol in a vessel in the form of the substance without a solvent,
b) mixing the solution, suspension or substance with fumaric acid, a suspension or a solution of fumaric acid in a solvent, optionally cooling and maintaining at low temperature,
c) isolating the compound of formula I,
wherein steps a) and b) can also be interchanged.

10. Pharmaceutical composition comprising an effective amount of 3-[2-(dimethylamino)methyl-(cyclohex-1-yl)]-phenol fumarate of formula I according to claim 1 and a pharmaceutical carrier or a pharmaceutical diluent.

11. Composition according to claim 10, wherein the compound of formula I is present in crystalline form A, crystalline form B or in a mixture of forms A and B.

12. Composition according to claim 11, wherein the compound of formula I is present as crystalline form B.

13. Use of 3-[2-(dimethylamino)methyl-(cyclohex-1-yl)]-phenol fumarate of formula I in the preparation of a pharmaceutical composition.

14. Use according to claim 13 in the preparation of a pharmaceutical composition for the treatment of pain.

## Revendications

1. Sels d'acide fumarique et de 3-[2-(diméthylamino)méthyl-(cyclohex-1-yl)]-phénol de formule I

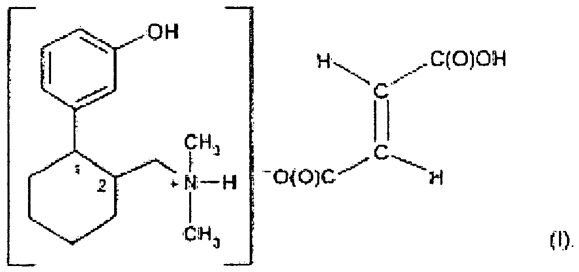

2. Sels selon la revendication 1, **caractérisés en ce que** le composé de formule I est présent sous forme de diastéréo-isomère ou sous forme de mélange de diastéréo-isomères énantiomères avec configuration trans du noyau phényle et du groupe diméthylaminométhyle (configuration 1R,2R ou configuration 1S,2S).

3. Sels selon la revendication 2, **caractérisés en ce que** le composé de formule I est présent sous forme d'énantiomère avec la configuration absolue (1R,2R).

4. Sels selon la revendication 1, **caractérisés en ce que** le fumarate de 3-[2-(diméthylamino)méthyl-(cyclohex-1-yl)]-phénol de formule I selon la revendication 1 est présent sous une forme cristalline B, qui :
présente une image de diffraction aux rayons X caractéristique dans la plage de 2° à 35° 2θ avec des lignes caractéristiques marquées, exprimées en valeurs d (Å) _{:} 9,3 (vs), 7,0 (m), 6,7 (s), 5,37 (s), 5,21 (s), 4,64 (s), 4,52 (s), 4,28 (vs), 4,23 (s), 4,19 (s), 3,94 (m), 3,78 (m), 3,52 (m), 3,49 (m), 3,33 (s), 3,30 (m), 3,06 (s), 2,83 (m).

5. Sels selon la revendication 4, **caractérisés en ce que** le fumarate de 3-[2-(diméthylamino)méthyl-(cyclohex-1-yl)]-phénol est présent dans la configuration (1R,2R).

6. Sels selon la revendication 1, **caractérisés en ce que** le fumarate de 3-[2-(diméthylamino)méthyl-(cyclohex-1-yl)]-phénol de formule I selon la revendication 1 est présent sous une forme cristalline A, qui :
présente une image de diffraction aux rayons X caractéristique dans la plage de 2° à 35° 2θ avec des lignes caractéristiques marquées, exprimées en valeurs d (Å) : 9,3 (vs), 7,0 (m), 6,7 (s), 5,36 (vs), 5,20 (m) 4,64 (vs), 4,53 (s), 4,26 (vs), 4,18 (s), 3,95 (m), 3,78 (m), 3,57 (m), 3,50 (s), 3,46 (m), 3,33 (s), 3, 05 (m), 2,83 (m).

7. Sels selon la revendication 6, **caractérisés en ce que** le fumarate de 3-[2-(diméthylamino)méthyl-(cyclohex-1-yl)]-phénol est présent dans la configuration (1R,2R).

8. Procédé de production du fumarate de 3-[2-(diméthylamino)méthyl-(cyclohex-1-yl)]-phénol de formule I selon la revendication 1, comprenant l'association de 3-[2-(diméthylamino)méthyl-(cyclohex-1-yl)]-phénol et d'acide fumarique.

9. Procédé selon la revendication 8 pour la production du fumarate de 3-[2-(diméthylamino)méthyl-(cyclohex-1-yl)]-phénol de formule I selon la revendication 1, comprenant les étapes suivantes :
a) dissolution ou suspension de 3-[2-(diméthylamino)méthyl-(cyclohex-1-yl)]-phénol dans un solvant, ou présentation de 3-[2-(diméthylamino)méthyl-(cyclohex-1-yl)]-phénol en substance,
b) mélange de la solution, suspension ou substance avec de l'acide fumarique, une suspension ou une solution d'acide fumarique dans un solvant, éventuellement refroidissement et maintien à basse température,
c) isolation du composé de formule I,
dans lequel les étapes a) et b) pouvent être également permutées.

10. Composition pharmaceutique, contenant une quantité efficace de fumarate de 3-[2-(diméthylamino)méthyl-(cyclohex-1-yl)]-phénol de formule I selon la revendication 1 et un support pharmaceutique ou un diluant pharmaceutique.

11. Composition selon la revendication 10, dans laquelle le composé de formule I est présent sous forme cristalline A, forme cristalline B ou sous forme d'un mélange des formes A et B.

12. Composition selon la revendication 11, dans laquelle le composé de formule I est présent sous forme cristalline B.

13. Utilisation du fumarate de 3-[2-(diméthylamino)méthyl-(cyclohex-1-yl)]-phénol de formule I pour la production d'une composition pharmaceutique.

14. Utilisation selon la revendication 13 pour la production d'une composition pharmaceutique pour le traitement d'états de douleur.
